# EUROPEAN PATENT SPECIFICATION

(11) **EP 3 087 172 B1**
(45) Date of publication and mention of the grant of the patent: **01.04.2020**
(21) Application number: 14821509.8
(22) Date of filing: 17.12.2014
(51) Int. Cl.: C12N 1/12

(54) **PALYTOXYN, ITS MEDICAL USE AND PROCESS FOR ITS ISOLATION**
PALYTOXYN, DESSEN MEDIZINISCHE VERWENDUNG UND VERFAHREN ZU DESSEN ISOLIERUNG
PALYTOXINE, SON USAGE MÉDICAL ET PROCÉDÉ POUR SON ISOLATION

(30) Priority: 17.12.2013 EP 13197854
(43) Date of publication of application: 02.11.2016
(73) Proprietor: Coral Biome, 13009 Marseille (FR); Institut de Recherche pour le Développement (IRD), 13572 Marseille Cedex 02 (FR)
(72) Inventor: DETOURNAY, Olivier, F-13190 Allauch (FR); LORQUIN, Jean, F-83330 le Beausset (FR); GAULT, Frederic, F-83330 Le Beausset (FR)
(74) Representative: Lavoix
(86) International application number: PCT/EP2014/003411
(87) International publication number: WO 2015/090591

(56) References cited:
- JAMES D REIMER ET AL: "Molecular identification of symbiotic dinoflagellates (Symbiodinium spp.) from Palythoa spp. (Anthozoa: Hexacorallia) in Japan", CORAL REEFS ; JOURNAL OF THE INTERNATIONAL SOCIETY FOR REEF STUDIES, SPRINGER, BERLIN, DE, vol. 25, no. 4, 14 September 2006 (2006-09-14), pages 521-527, XP019459567, ISSN: 1432-0975, DOI: 10.1007/S00338-006-0151-4
- R. J. QUINN ET AL: "Anticancer activity of zoanthids and the associated toxin, palytoxin, against ehrlich ascites tumor and P-388 lymphocytic leukemia in mice", JOURNAL OF PHARMACEUTICAL SCIENCES, vol. 63, no. 2, 1 February 1974 (1974-02-01), pages 257-260, XP055113357, ISSN: 0022-3549, DOI: 10.1002/jps.2600630217
- JAMES DAVIS REIMER ET AL: "Species Diversity of Shallow Water Zoanthids (Cnidaria: Anthozoa: Hexacorallia) in Florida", JOURNAL OF MARINE BIOLOGY, vol. 10, no. 5114, 1 January 2012 (2012-01-01), pages 57-14, XP055113398, ISSN: 1687-9481, DOI: 10.1046/j.1365-294X.2002.01652.x
- HIDESHI NAKAMURA ET AL: "Studies on Polyketide Metabolites of a Symbiotic Dinoflagellate, Symbiodinium sp", BULLETIN OF THE CHEMICAL SOCIETY OF JAPAN, vol. 71, no. 4, 1 January 1998 (1998-01-01), page 781, XP055113408, ISSN: 0009-2673
- BERESS L ET AL: "A method for the isolation of the caribbean palytoxin (C-PTX) from the coelenterate (zooanthid) Palythoa caribaeorum", TOXICON, ELMSFORD, NY, US, vol. 21, no. 2, 1 January 1983 (1983-01-01), pages 285-290, XP025508816, ISSN: 0041-0101, DOI: 10.1016/0041-0101(83)90013-2 [retrieved on 1983-01-01] cited in the application
- NAKAMURA H ET AL: "Isolation of zooxanthellatoxins, novel vasoconstrictive substances from the zooxanthella Symbiodinium sp", TOXICON, ELMSFORD, NY, US, vol. 31, no. 4, 1 April 1993 (1993-04-01), pages 371-376, XP025828057, ISSN: 0041-0101, DOI: 10.1016/0041-0101(93)90172-F [retrieved on 1993-04-01]
- JONATHAN R. DEEDS ET AL: "Palytoxin Found in Palythoa sp. Zoanthids (Anthozoa, Hexacorallia) Sold in the Home Aquarium Trade", PLOS ONE, vol. 6, no. 4, 1 January 2011 (2011-01-01) , page e18235, XP055113680, ISSN: 1932-6203, DOI: 10.1371/journal.pone.0018235 cited in the application

## Description

The present international patent application claims the priority of the European patent application n°13197854.6 filed on December 17, 2014.

### Domain of the invention

The invention relates to a pharmaceutical composition comprising palytoxin for use for preventing and/or treating leukaemia. The invention also discloses a method for obtaining palytoxin (PLTX) from *Palythoa* aff. *clavata,* methods for isolating the *Symbiodinium* dinoflagellate from *Palythoa* aff. *clavata* and discloses a method for producing PLTX from the *Symbiodinium* dinoflagellate. The invention also discloses the use of PLTX for the prevention and/or the treatment of cancers at infinitesimal doses, *i.e.* 10⁻¹² mol.L⁻¹, and for the prevention and/or the treatment of diseases and/or disorders chosen amongst nosocomial diseases, parasitic diseases (*Plasmodium falciparum, Leishmanii*), Human Immunodeficiency Virus (HIV) disease, Ebola Virus Disease (EVD), and skin diseases (*psoriasis, Propionibacterium acnes*).

### Background of the invention

Leukaemia is a cancer of the blood cells.

Clinically and pathologically, leukaemia is subdivided into a variety of large groups. The first division is between its acute and chronic forms:
- Acute leukaemia is characterized by a rapid increase in the number of immature blood cells. Crowding due to such cells makes the bone marrow unable to produce healthy blood cells. Immediate treatment is required in acute leukaemia due to the rapid progression and accumulation of the malignant cells, which then spill over into the bloodstream and spread to other organs of the body. Acute forms of leukaemia are the most common forms of leukaemia in children.
- Chronic leukaemia is characterized by the excessive buildup of relatively mature, but still abnormal, white blood cells. Typically taking months or years to progress, the cells are produced at a much higher rate than normal, resulting in many abnormal white blood cells. Whereas acute leukaemia must be treated immediately, chronic forms are sometimes monitored for some time before treatment to ensure maximum effectiveness of therapy. Chronic leukaemia mostly occurs in older people, but can theoretically occur in any age group.

Additionally, the diseases are subdivided according to which kind of blood cell is affected. This split divides leukaemias into lymphoblastic or lymphocytic leukaemias and myeloid or myelogenous leukaemias:
- In lymphoblastic or lymphocytic leukaemias, the cancerous change takes place in a type of marrow cell that normally goes on to form lymphocytes, which are infection-fighting immune system cells. Most lymphocytic leukaemias involve a specific subtype of lymphocyte, the B cell.
- In myeloid or myelogenous leukaemias, the cancerous change takes place in a type of marrow cell that normally goes on to form red blood cells, some other types of white cells, and platelets.

Combining these two classifications provides a total of four main categories.

Now, new therapeutic treatment are still needed for treating such leukaemia.

### Summary of the invention

Palytoxin, also called PLTX, is a nonprotein toxin of marine origin (Wiles *et al.,* 1974).

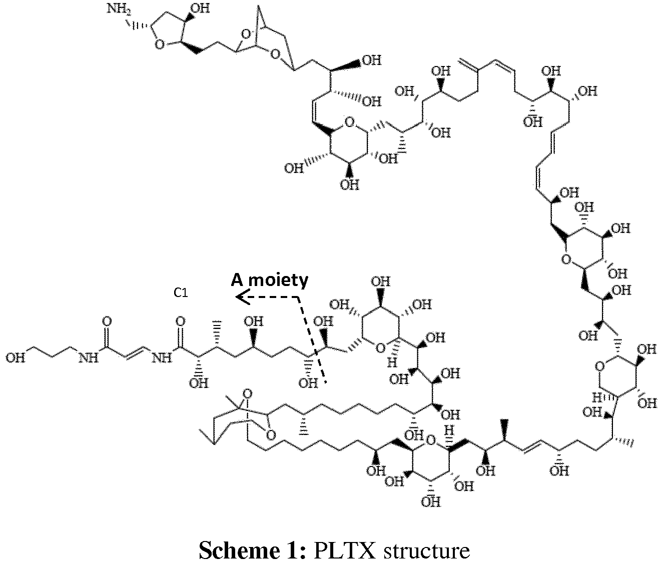

Even though PLTX was originally found in soft corals from tropical areas of the Pacific Ocean, *i.e.* in Hawaii from the tropical soft coral *Palythoa* sp., a zoanthid (Moore and Scheuer, 1971), its occurrence was then observed in numerous other marine organisms from the same ecological region. The literature emphasizes the sodium/potassium pump (the Na⁺/K⁺-ATPase) as the privileged target for PLTX when exerting its cytotoxic/toxic effects. Recently several analogs of PLTX were discovered in various species of the dinoflagellate genus *Ostreopsis* (Ramos and Vasconcelos, 2010). Indeed, sporadic occurrence of PLTX in algae, crabs and fish alike indicates that microorganisms could represent the primary source of these toxins. **Table 1** below gives a non exhaustive summary of the PLTX analogues and their respective marine sources.

**Table 1: Summary of the known molecules in the PLTX family in zoantharians, cyanobacteria, algae and dinoflagellates**

| **Compounds** | **Organism** | **Sample IDMW** | | **Concentration** | **References** |
|---|---|---|---|---|---|
| | **Zoantharians** | | | | |
| PLTX | *Palythoa toxica* | | 2679 | 275 µg/g wet z | Moore and Scheuer, 1971 |
| PLTX | *Palythoa tuberculosa* | | 2679 | 13.6 µg/g wet z | Kimura and Hashimoto, 1973 |
| PLTX | *Palythoa caribaeorum* | | 2679 | 30 µg/g dry z | Beress *et al.,* 1983 |
| PLTX | *Palythoa heliodiscus* | VAZOA | 2679 | 613 µg/g wet z | Deeds *et al.,* 2011 |
| PLTX | *Palythoa heliodiscus* | 305.11.2 | 2679 | 515 µg/g wet z | Deeds *et al.,* 2011 |
| PLTX | *Palythoa heliodiscus* | 306.39.2 | 2679 | 116 µg/g wet z | Deeds *et al.,* 2011 |
| PLTX | *Palythoa heliodiscus* | 306.39.3 | 2679 | 1037 µg/g wet z | Deeds *et al.,* 2011 |
| PLTX | *Palythoa vestitus* | | 2674 | ND | Quinn *et al.,* 1974 |
| PLTX | *Palythoa* aff. *margaritae* | | 2674 | ND | Oku *et al.,* 2004 |
| PLTX | *Zoanthus solanderi* | | 2674 | ND | Gleibs *et al.,* 1995 |
| PLTX | *Zoanthus sociatus* | | 2674 | ND | Gleibs *et al.,* 1995 |
| PLTX-b | *Palythoa tuberculosa* | | 2720 | minor | Rossi *et al.,* 2010 |
| Homo-PLTX | *Palythoa tuberculosa* | | 2692 | minor | Uemura *et al.,* 1985 |
| Bishomo-PLTX | *Palythoa tuberculosa* | | 2706 | minor | Uemura *et al.,* 1985 |
| Neo-PLTX | *Palythoa tuberculosa* | | 2661 | minor | Uemura *et al.,* 1985 |
| Deoxy-PLTX | *Palythoa tuberculosa* | | 2662 | minor | Uemura *et al.,* 1985 |
| Deoxy-PLTX | *Palythoa heliodiscus* | 306.37.3 | 2679 | 3515 µg/g wet z* | Deeds *et al.,* 2011 |
| 42-Hydroxy-PLTX | *Palythoa tuberculosa* | | 2694 | minor | Ciminiello *et al.,* 2009 |
| 42-Hydroxy-PLTX | *Palythoa toxica* | | 2694 | minor | Ciminiello *et al.,* 2009 |

| | **Cyanobacteria** | | | | |
|---|---|---|---|---|---|
| 42-Hydroxy-PLTX | *Trichodesmium* spp. | | 2694 | minor | Kerbrat *et al.,* 2011 |

| | **Algae** | | | | |
|---|---|---|---|---|---|
| CA-I | *Chondria armata* | | | ND | Yasumoto and Murata, 1990 |
| CA-II | *Chondria armata* | | | ND | Yasumoto and Murata, 1990 |

| | **Dinoflagellates** | | | | |
|---|---|---|---|---|---|
| Mascarenotoxin-a | *Ostreopsis mascarenensis* | | 2588 | minor | Lenoir *et al.,* 2004 |
| Mascarenotoxin-a | *Ostreopsis ovata* | | 2588 | minor | Rossi *et al.,* 2010 |
| Mascarenotoxin-b | *Ostreopsis mascarenensis* | | 2606 | minor | Lenoir *et al.,* 2004 |
| Mascarenotoxin-c | *Ostreopsis ovata* | | 2628 | minor | Rossi *et al.,* 2010 |
| Ostreocin-d | *Ostreopsis siamensis* | | 2634 | ND | Ukena *et al.,* 2001 |
| Ovatoxin-a | *Ostreopsis ovata* | | 2646 | minor | Ciminiello *et al.,* 2008 |
| Ovatoxin-b | *Ostreopsis ovata* | | 2662 | minor | Rossi *et al.,* 2010 |
| Ovatoxin-c | *Ostreopsis ovata* | | 2690 | minor | Rossi *et al.,* 2010 |
| Ovatoxin-d | *Ostreopsis ovata* | | 2706 | minor | Rossi *et al.,* 2010 |

| | | | | | |
|---|---|---|---|---|---|
| ND, not determined; z, zoanthid.*, position of the deoxygenation was not determined for this analogue. | | | | | |

In most cases, the yield of extraction/isolation is low, pointing out the need to develop solutions to obtain higher level of toxin. Indeed, large quantity of such a molecule is required for its functional characterization applied, for instance, to cancer research. Nowadays, Wako Pure Chemical Industries in Japan remains the unique PLTX supplier in the world, their toxin being purified from *Palythoa caribaeorum* (Béress *et al.,* 1983; see **Table 1**).

PLTX has a very complex structure (**Scheme 1**) with a long polyhydroxylated and partially unsaturated aliphatic backbone containing 64 chiral centers (Kan *et al.,* 2001). It is heat-stable, not inactivated by boiling, and stable in neutral aqueous solutions for prolonged periods. In contrast, a rapid degradation occurs under acid or alkaline conditions, leading to loss of its toxicity (Katikou, 2007). Resembling to mycotoxins, other polyketide-type molecules, no pharmacological treatment have been developed to fight and/or destroy PLTX in the human or animal body.

Most of the data published in the literature about PLTX toxicity relates to PLTX mediated disruption of the actin cytoskeleton (Louzao *et al.,* 2008 and 2011). The widely accepted molecular action is blockade of the Na⁺/K⁺-ATPase pump (NaK) (Hilgeman, 2003; Rodrigues *et al.,* 2008; Charlson *et al.,* 2009; Rossini and Bigiani, 2011; Wattenberg, 2011). PLTX seems to bind to the extracellular part of the NaK and thereby inhibits the active transport of Na⁺ and K⁺ across the cell membrane by transforming the pump into a nonspecific permanently open ion channel (Ramos and Vasconcelos, 2010). The membrane depolarization generated and the massive increase of Ca²⁺ in the cytosol (Satoh *et al.,* 2003) interferes with some vital functions of cells. It has recently been demonstrated that PLTX acts through voltage-dependent channels and the Na⁺/Ca²⁺ exchanger (reverse mode) (Del Favero *et al.,* 2012), emphasizing at best that the NaK would not be its only cellular target. PLTX has also been identified as a carcinogenic agent (Wattenberg, 2011). Thus, the identification of PLTX as a tumor promoter, together with the recognition that the Na⁺/K⁺-ATPase is its receptor, suggest that PLTX triggers the modulation of signal transduction pathways. In fact, mitogen activated protein (MAP) kinases are mediators of PLTX-stimulated signaling and relay a variety of signals to the cellular machinery that regulates cell fate and function (Wattenberg, 2011).

If a cytotoxic effect for PLTX was identified in different articles and for different tumor cell lines, said activity was very dependent upon the cancer target cells as disclosed in BELLOCI *et al.* (2011). As an example, QUINN *et al.* (1974) in addition to established an antitumoral activity on mammary adenocarcinoma, also show that PLTX has only a marginal activity on leukaemia.

Now, the inventors have surprisingly established 40 years later that PLTX has finally a strong a reproducible antitumoral activity on all tested leukaemia cell lines, including both lymphoblastic and myelogenous leukaemia.

The P-388 cell lines identified by DAWE & POTTER (1957) is a murine cell lines corresponding to one of the first model of leukaemia. Now, the correlation between drugs active against P388 leukaemia and solid experimental tumor models has not been good. One common observation is that some drugs that are active against experimental solid tumors are inactive against P388 leukaemia. For example, 15% of 84 agents that were inactive against P388 leukaemia were active against at least one of eight solid tumors tested (STAQUET *et al.,* 1983).

Following the results obtained by the inventors on human leukaemia cell lines, it seems finally that the results of QUINN *et al.* (1974) with PLTX on murine P388 cell lines are not representative of the mechanism of PLTX on leukaemia. QUINN *et al.* evaluated the anticancer activity of zoanthids and the associated toxin, palytoxin, against ehrlich ascites tumor and P-388 lympthocytic leukaemia in mice.

Thus, the invention is as defined in the claims and in particular directed to a pharmaceutical composition for use in the prevention and/or treatment of leukaemia in a subject, said composition comprising palytoxin (PLTX) and a pharmaceutically acceptable carrier.

The Inventors have also identified a new PLTX producer giving the highest yield ever found in the nature. The Inventors have actually found that the extraction from invertebrate *Palythoa* aff. *clavata* species which live in symbiosis with a *Symbiodinium* dinoflagellate from the C clade allows to obtain a pure PLTX with excellent yields, thanks to a simple and economic process (limited number of operating steps).

Therefore, it is disclosed a method for obtaining pure PLTX from *Palythoa* aff. *clavata* polyps, said polyps comprising the *Symbiodinium* dinoflagellate and possibly other symbiotic microorganisms. The method for obtaining pure PLTX may comprise the following steps:
(i) immersion of *Palythoa* aff. *clavata* polyps in a polar solvent, under stirring,
(ii) decantation of the mixture obtained from step (i),
(iii) filtration and/or centrifugation of the liquid obtained from step (ii), and
(iv) evaporation of the solvent, preferably by rotary evaporation under reduced air pressure,
(v) purification of the pellet obtained from step (iv), preferably by reversed phase liquid chromatography, and more preferably on a column Lichroprep® RP18,
(vi) evaporation to dryness of the pellet obtained from step (v), preferably by rotary evaporation and then under reduced N₂ pressure.

### Brief description of the figures

- **Figure 1** (**A**) and (**B**) are pictures of a Zoanthid colony collected in Indo-Pacific and cultivated at Coral Biome aquarium,
- **Figure 2** represents the HPLC chromatogram of the purified PLTX from *Palythoa* aff. *clavata* at 263 nm. The insert graph is the UV spectrum of PLTX eluted at 14.8 min in the gradient conditions used,
- **Figure 3** represents the MALDI-ToF mass spectrum of PLTX purified from *Palythoa* aff. *clavata,*
- **Figure 4** shows the maximum likelihood rooted tree for the dataset based on 34 ITS-rDNA sequences of Zoanthidae species (937 nucleotide positions). One hundred heuristic replicates were performed using the Kimura's 2-parameters model with estimation of gamma parameter shape distribution and proportion of invariant sites (K2 + G5 + I). Bootstrap values are shown at nodes with values > 75%. Black arrowhead indicates the sequence belonging to the *Palythoa* species used for PLTX extraction. Sequences/species names from previous studies in regular font with GenBank Accession Numbers,
- **Figure 5** shows the maximum likelihood rooted tree for the dataset based on 30 COI sequences of Zoanthidae species (462 nucleotide positions). One hundred heuristic replicates were performed using the Kimura's 2-parameters model (K2). Bootstrap values are shown at nodes with values > 50%. Black arrowhead indicates the sequence belonging to the *Palythoa* species used for PLTX extraction. Sequences/species names from previous studies in regular font with GenBank Accession Numbers,

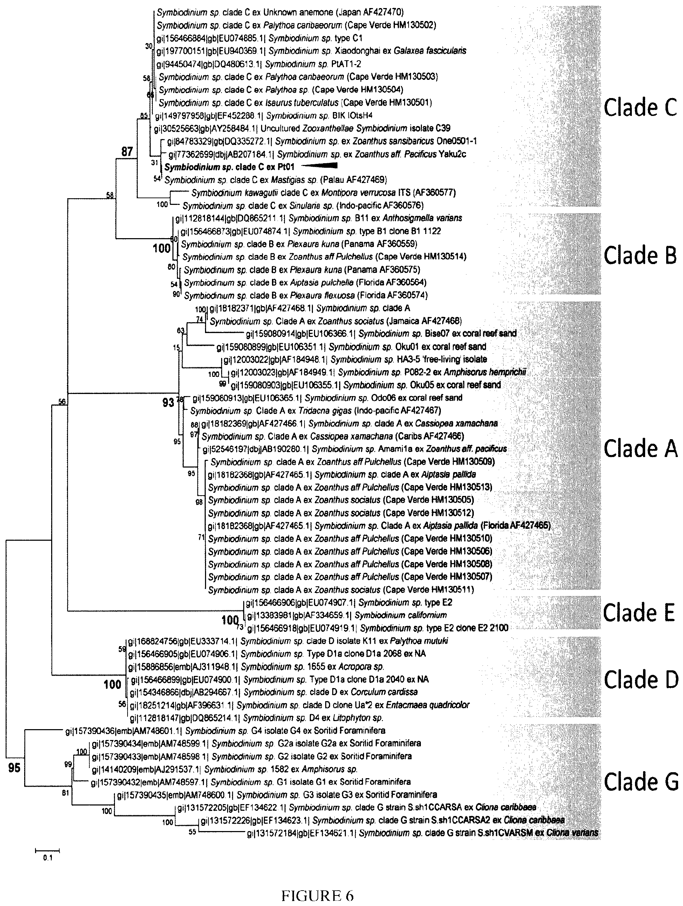

- **Figure 6** shows the maximum likelihood unrooted tree based on the internal transcribed spacer 2 of ribosomal DNA (ITS2-rDNA) sequences for *Symbiodinium* from various corals and molluscs, including the *Palythoa* specimen from this study (332 nucleotide positions). One hundred replicates were performed using the Kimura's 2-parameters model with estimation of gamma parameter shape distribution (K2 + G5). Values at branches represent ML bootstrap values. New sequence from this study in bold. Sequences/species names from previous studies in regular font with GenBank Accession Numbers, species-associated isolates from previous studies with species name and location, as well as GenBank Accession Number. On the right, *Symbiodinium* clades confirmed to be in symbiosis with various metazoans species designated with shaded boxes,
- **Figure 7** represents the MTT colorimetric assay results visualized by the remaining viable cells versus PLTX concentration. (**A**) Experiences performed with the cancer and the normal but transformed cells, HBL-100. (**B**) With the normal and human cell line MSC. (C) With the normal and human cell line NHDF,
- **Figure 8** represents the videomicroscopy experiences on human Hs683 oligodendroglioma cells (apoptose-sensitive) in presence of 0.01 or 1 nM PLTX during 22 h,
- **Figure 9** represents the videomicroscopy experiences on human U373n glioblastoma cells (apoptose-sensitive) in presence of 0.01 or 1 nM PLTX during 22 h.

### Detailed description of the invention

The invention is directed to a pharmaceutical composition for use in the prevention and/or treatment of leukaemia in a subject, said composition comprising palytoxin (PLTX) and a pharmaceutically acceptable carrier.

As used herein, the term "subject" denotes a mammal, such as a rodent, a feline, a canine or a primate, and most preferably a human.

The expression "pharmaceutically acceptable" refers to molecular entities and compositions that are physiologically tolerable and do not typically produce allergic or similar undesirable reactions, such as gastric upset, dizziness and the like when administered to a human. Preferably, as used herein, the expression "pharmaceutically acceptable" means approvable by a regulatory agency of the Federal or state government or listed in the U.S. Pharmacopeia or other generally recognized pharmacopeia for use in animals, and more particularly in humans.

The term "carrier" refers to a solvent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like.

The route of administration is preferably parenteral; as used herein, the term "parenteral" includes intravenous, intramuscular, subcutaneous, rectal, vaginal or intraperitoneal administration. Thus, the pharmaceutical composition contains vehicles which are pharmaceutically acceptable for a formulation intended to be injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. Suitable pharmaceutical carriers are described in "Remington's Pharmaceutical Sciences" by E.W. Martin. Of these, intravenous and subcutaneous administration are most preferred.

PLTX may be solubilized in a buffer or water or incorporated in emulsions, microemulsions, hydrogels (e.g. PLGA-PEG-PLGA triblock copolymers-based hydrogels), in microspheres, in nanospheres, in microparticles, in nanoparticles (e.g. poly(lactic-co-glycolic acid) microparticles (e.g. poly lactic acid (PLA) ; poly (lactide-co-glycolic acid) (PLGA) ; polyglutamate microspheres, nanospheres, microparticles or nanoparticles), in liposomes, or other galenic formulations. In all cases, the formulation must be sterile and fluid to the extent of acceptable syringability. It must be stable under the conditions of manufacture and storage and must be preserved against the contaminating action of microorganisms, such as bacteria and fungi.

Dispersions can also be prepared in glycerol, liquid polyethylene glycols, and mixtures thereof and in oils. Under ordinary conditions of storage and use, these preparations contain a preservative to prevent the growth of microorganisms.

The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride.

Prolonged absorption of the injectable compositions can be brought about by the use in the compositions of agents delaying absorption, for example, aluminium monostearate, gelatin, polyols, half-life enhancing covalent and non covalent formulations.

In the context of the invention, the term "treatment", as used herein, means reversing, alleviating, inhibiting the progress of the disorder or condition to which such term applies, or one or more symptoms of such disorder or condition.

The term "treatment of leukaemia" as used herein means the inhibition of the growth of leukaemia cells. Preferably such treatment also leads to the regression of tumor growth, i.e., the decrease in size of a measurable tumor. Most preferably, such treatment leads to the complete regression of the tumor.

As used herein, the term "leukaemia" refers to lymphoblastic and/or myelogenous leukaemia. The term "leukaemia" refers to acute and/or chronic leukaemia.

An "effective amount" of palytoxin is an amount which is sufficient to induce the regression of tumor growth. The doses used for the administration can be adapted as a function of various parameters, in particular as a function of the mode of administration used, of the relevant pathology, or alternatively of the desired duration of treatment. Naturally, the form of the pharmaceutical composition, the route of administration, the dosage and the regimen naturally depend on the condition to be treated, the severity of the illness, the age, weight, and sex of the subject, etc. The ranges of effective doses provided below are not intended to limit the invention and represent preferred dose ranges. However, the preferred dose can be tailored to the individual subject, as is understood and determinable by one of skill in the art, without undue experimentation.

As an illustration, an effective amount of PLTX is comprised between 10 ng/kg and 2 µg/kg, preferably between 20 ng/kg and 1 µg/kg, and most preferably between 50 ng/kg and 0.5 µg/kg. Other dosages are viable, since the molecular weight and the activity of the conjugate thereof may influence it. The skilled artisan is readily credited with determining a suitable dosage that falls within the ranges, or if necessary, outside of the ranges.

Still preferably, said effective amount corresponds to a daily effective amount.

The used PLTX may be obtained from different sources. As an example, such PLTX may be obtained from *Palythoa* aff. *clavata* polyps, or obtained from the culture of the *Symbiodinium* dinoflagellate isolated from *Palythoa* aff. *Clavata.* Such PLTX may be obtained by the method described hereafter.

It is also disclosed a method for obtaining pure PLTX from *Palythoa* aff. *clavata* polyps, said polyps comprising the *Symbiodinium* dinoflagellate and possibly other symbiotic microorganisms. The method for obtaining pure PLTX may comprise the following steps:
(i) immersion of *Palythoa* aff. *clavata* polyps in a polar solvent, under stirring,
(ii) decantation of the mixture obtained from step (i),
(iii) filtration and/or centrifugation of the liquid obtained from step (ii), and
(iv) evaporation of the solvent, preferably by rotary evaporation under reduced air pressure,
(v) purification of the pellet obtained from step (iv), preferably by reversed phase liquid chromatography, and more preferably on a column Lichroprep® RP18,
(vi) evaporation to dryness of the pellet obtained from step (v), preferably by rotary evaporation and then under reduced N₂ pressure.

In the sense of the invention, a polyp is a coral that has typically a hollow cylindrical body closed and attached at one end and opening at the other by a central mouth surrounded by tentacles armed with nematocysts.

Step (i) is preferably carried out at a temperature ranging from 4 to 10°C, more preferably from 4 to 6°C, under stirring. The stirring can be maintained during 5 to 15 hours, and preferably during 10 hours. According to a preferred embodiment, step (i) is implemented in the dark.

The polar solvent of step (i) may be a mixture of alcohol and water, and is preferably a mixture of methanol or ethanol with water. More preferably said solvent is a methanol/water mixture, more preferably in a ratio 80/20.

According to a preferred embodiment, step (iii) is a filtration step carried out on a PTFE membrane, preferably having a pore size ranging from 0.45 to 3.0 µm, or carried out by centrifugation (8000 g, 20 min). For a large-scale production, filtration would be preferred.

After homogenisation of the pellet with a mortar, the resulting powder may be diluted in cold milli-Q water and purified, preferably on a mini-RP18 solid phase, and more preferably on a Lichroprep® RP₁₈ column.

According to another embodiment, the method for obtaining PLTX may comprise a subsequent liquid-liquid extraction step between the evaporation step (iv) and the purification step (v), preferably with hexane or dichloromethane.

The method for obtaining pure PLTX then comprise a purification step (v), preferably by reversed phase liquid chromatography. The purification step (v) may be implemented on a mini-RP18 solid phase, preferably on a Lichroprep® RP₁₈ column.

The method for obtaining pure PLTX may also comprise a preliminary treatment of the *Palythoa* aff. *clavata* by liquid nitrogen directly pooled on the dried out (paper) and chopped (scalpel) polyps.

*Symbiodinium* is a highly diverse taxon of dinoflagellates (or zooxanthellae) with nine clades (A-I) described to date and with the phylogenetic distance between clades at the order or family level (Coffroth and Santos, 2005; Pochon and Gates, 2010). In addition to the well-known symbiotic clades A-D, microbial ecologists are now describing clades that are likely only free-living and not in symbiosis with host animals (Apprill and Gates, 2007; Pochon and Gates, 2010). Patterns of host-symbiont specificity are highly complex and influenced by a broad array of factors including biogeography, environment, stress, and host ontogeny (Baker, 2003; Coffroth and Santos, 2005). *Symbiodinium* types have highly varying biochemistry, physiology, and responses to environmental stress (Iglesias-Prieto and Trench, 1997; Warner *et al.,* 1999; Rowan, 2004; Tchernov *et al.,* 2004; Stat *et al.,* 2008). These differential biochemical and physiological qualities have direct impacts on the performance and health of the holobiont (or partnership). An obvious example is the differential performance of stress-sensitive clade C *versus* stress-resistant clade D symbionts to elevated temperature in corals. Whereas clade C symbionts are the overwhelmingly dominant taxon in healthy IndoPacific corals, they are at least temporarily displaced after bleaching events by clade D types in many coral species (Berkelmans and van Oppen, 2006; Stat and Gates, 2011).

PLTX biosynthesis by the symbiotic *Symbiodinium* dinoflagellate represents a reproducible way to increase the yield of production at lower cost, as already suggested (Taniyama *et al.,* 2003; Katikou, 2007). The Inventors have shown that the *Symbiodinium* ITS2-rDNA sequence isolated from the *Palythoa* aff. *clavata* was unambiguously placed within the generalist clade C, within a large group including many subclades of C previously observed in a variety of hosts.

Several techniques have been developed to isolate the symbiotic algae from the host. They include stripping coral tissues from the skeleton with a fine jet of seawater using a WaterPik (Johannes and Wiebe, 1970), grinding the whole coral (Santiago-Vázquez *et al.,* 2006) or gorgonian tissue (Forcioli *et al.,* 2011; Pey *et al.,* 2011) in liquid nitrogen, manual tissue fractionation and potter homogenization (Richier *et al.,* 2003). Although these techniques are effective, the *Symbiodinium* isolation is complicated, and the dinoflagellate not purified. Furthermore, the molecular analysis leads to DNA extracts containing both cnidarian and algal genomes. These techniques however remain valuable and could be used as comparative techniques. As an improved method, a fast and efficient protocol without host contaminant and developed on three cnidarian species has been recently validated (Zamoum and Furla, 2012).

Therefore, it is also disclosed the production of PLTX from the culture of symbiotic dinoflagellate. The Inventors have now found new methods for isolating the *Symbiodinium* dinoflagellate from *Palythoa* aff. *clavata* polyps, as well as a method for producing PLTX by culture of the isolated *Symbiodinium* dinoflagellate, said methods simplifying the culture and the PLTX extraction, and being time-saving.

The Inventors have thus developed a method for isolating the *Symbiodinium* dinoflagellate from *Palythoa* aff. *clavata* polyps, comprising the following steps:
(i') suspension of *Palythoa* aff. *clavata* polyps in an extraction buffer, preferably a phosphate buffer,
(ii') centrifugation of the mixture obtained from step (i'),
(iii') washing the pellet obtained from step (ii') in an extraction buffer, preferably a phosphate buffer,
(iv') grounding the pellet obtained from step (iii') with liquid nitrogen,
(v') resuspension of the powder obtained from step (iv') in an extraction buffer, preferably a phosphate buffer,
(vi') filtration of the liquid obtained from step (v'), and
(vii') isolation of the *Symbiodinium* colonies.

The *Palythoa* aff. *clavata* polyps may be dried out and chopped into pieces before being suspended in the extraction buffer in step (i'). The suspension of step (i') may be homogenized, for example by sonication or with a syringe.

Preferably, the extraction buffer is a phosphate buffer of pH 7.8 having a concentration ranging from 25 to 75 mM, and preferably of 50 mM. Said phosphate buffer may be mixed with sorbitol to prevent the *Symbiodinium* dinoflagellate from a membrane disruption.

The *Symbiodinium* dinoflagellate in the mixture of step (i') is separated by centrifugation during a step (ii').

Then, the pellet obtained from step (ii') is washed with an extraction buffer, and may be centrifuged again, and resuspended in the same extraction buffer. The extraction buffer is preferably a phosphate buffer. The pellet obtained from step (iii') is then ground, preferably in a mortar, with liquid nitrogen (step (iv')).

The powder obtained from step (iv') is then resuspended in an extraction buffer.

The liquid obtained from step (v') is then filtered during step (vi'), preferably through a nylon mesh (100 µm) in order to eliminate as many skeleton residues as possible.

The *Symbiodinium* colonies are isolated during a step (vii'), preferably on agar Petri dishes, and more preferably on 2.0% agar Petri dishes. Another method for isolating the *Symbiodinium* dinoflagellate from *Palythoa* aff. *clavata* has also been finalized by the Inventors, said method comprising the following steps:
(i") incubation of *Palythoa* aff. *clavata* polyps in a strong base, preferably NaOH, and
(ii") centrifugation of the mixture obtained from step (i"),
(iii") washing of the pellet obtained from step (ii"), preferably with water,
(iv") neutralization of the mixture obtained from step (iii") with a strong acid, preferably HCl,
(v") resuspension of the pellet obtained from step (iv"), preferably in water or in a medium containing seawater, and
(vi") isolation of the *Symbiodinium* colonies.

According to a preferred embodiment, the strong base implemented in step (i") has a concentration ranging from 0.25 to 10 mol.L⁻¹, preferably a concentration of 1 M.

The incubation step (i") may be carried out at a temperature ranging from 20 to 40°C, preferably at 37°C. The incubation step (i") may be carried out during 1 to 4 h, preferably 1 h.

The samples can be shaken vigorously every 15 min during the step (i").

The incubated *Palythoa* aff. *clavata* polyps are then separated during a centrifugation step (ii").

The pellet obtained from step (ii") is then washed with water during step (iii"), and preferably washed twice with milli-Q water.

The mixture obtained from step (iii") is then neutralized with a strong acid during a step (iv"), preferably HCl, and more preferably with a solution of 1 M HCl.

The pellet obtained from step (iv") is resuspended, for example in water (sterilized milli-Q water) or in a medium containing seawater such as a F/2 culture medium.

The F/2 culture medium is a common and widely used general enriched seawater medium designed for growing coastal marine algae, especially diatoms (Guillard and Ryther, 1962). For one Liter of natural seawater, the composition of the medium comprises (final concentration): NaNO₃ 882 µM, NaH₂PO₄ H₂O 36 µM, and 1 mL of trace metal solution. The solution is further autoclaved at 120°C for 20 min, and after reaching ambient temperature, 0.5 mL of vitamin solution is added under sterile conditions. The trace metal solution comprises (for one Liter of milli-Q water): FeCl₃ 6H₂O 3.15 g, Na₂EDTA 2H₂O 4.36 g, CuSO₄ 5H₂O 9.8 mg, Na₂MoO₄ 2H₂O 6.3 mg, ZnSO₄ 7H₂O 22.0 mg, CoCl₂ 6H₂O 10.0 mg, MnCl₂ 4H₂O 180 mg. The trace metal solution is then autoclaved at 120°C for 20 min and stored at 4°C. The vitamin solution comprises (for 1 Liter of milli-Q water): thiamine-HCl (vit. B₁) 200 mg, biotin (vit. H) 1 mg. The vitamin solution is then sterilized by passing on a 0.22 µm pore size filter (from Millipore) and stored at 4°C.

The *Symbiodinium* colonies are then isolated during a step (vi"), preferably on agar Petri dishes, and more preferably on 2.0% agar Petri dishes in the F/2 culture medium. In this case, 2.0% (w/v) of agar are added in the F/2 culture medium comprising NaNO₃ 882 µM, NaH₂PO₄ H₂O 36 µM, and 1 mL of trace metal solution. The solution is further autoclaved at 120°C for 20 min, and the vitamin solution is added on the Petri dishes at ambient temperature.

During the isolation step (vi"), the Petri dishes are also incubated, preferably at 26°C under a 12 h light/12 h dark photoperiod, until visualization of the *Symbiodinium* colonies.

Another object of the invention is a method for producing PLTX from a culture of the isolated *Symbiodinium* dinoflagellate, said method comprising the following steps:
(i''') cultivation of the *Symbiodinium* dinoflagellate in a F/2 culture medium, and
(ii''') irradiation of the *Symbiodinium* dinoflagellate culture of step (i''') under a photon flux ranging from 50 to 200 µmol.m⁻²s⁻¹.

According to a preferred embodiment, the incubation step (i''') is implemented at pH 8.2 and at a temperature of 26.0 ± 0.1°C. The composition of the F/2 culture medium of step (i''') is the same as described above.

According to another preferred embodiment, the irradiation step (ii''') is implemented at a photon flux of 100 µmol.m⁻².s⁻¹, during 12 h. Preferably, the irradiation step (ii''') is performed with a Sylvania Gro-Lux bulb.

In a subsequent step (iii'''), the irradiated *Symbiodinium* dinoflagellate culture obtained from step (i''') is centrifuged, preferably at 3000-5000 g, and more preferably at 3000 g. Preferably the centrifugation step (iii''') is performed at a temperature ranging from 4 to 10°C, and more preferably during 5 to 20 min.

During an optional step (iv'''), the pellet obtained from step (iii''') is resuspended with water, preferably ice-cold distilled water, and eventually further homogenized by sonication.

Then, the mixture obtained from step (iv''') may be separated during a centrifugation step (v'''), preferably at 10000-12000 g, and more preferably at 12000 g during 5 to 20 min. Alternatively, the mixture from step (iv''') may be filtered on a nylon mesh (1 µm pore size) so as to separate the debris from the PLTX.

The PLTX present in the filtrate can also be purified during a purification step (vi'''), preferably by reversed phase liquid chromatography. The purification step (vi''') may be implemented on a Lichroprep® RP₁₈ column.

The Inventors have also demonstrated that the PLTX obtained from *Palythoa* aff. *clavata* polyps is very efficient against cancer cell lines, with an IC₅₀ of 0.545 ± 0.05 pM, thus at lower doses than those known from the prior art (Quinn *et al.,* 1974; Valverde *et al.,* 2008; Görögh *et al.,* 2013; Pelin *et al.,* 2013). Therefore, the invention also discloses the use of pure PLTX obtained from *Palythoa* aff. *clavata* polyps, or obtained from the culture of the *Symbiodinium* dinoflagellate isolated from *Palythoa* aff. *clavata,* for its use for preventing and/or treating cancer at infinitesimal doses, *i.e.* at a dose equal or less than 10⁻¹² mol.L⁻¹.

It is also described the use of PLTX for the prevention and/or the treatment of diseases and/or disorders chosen amongst nosocomial diseases, parasitic diseases (*Plasmodium falciparum, Leishmanii*), Human Imminodeficiency Virus (HIV) disease, Ebola Virus Disease (EVD), skin diseases (*psoriasis, Propionibacterium acnes*).

In the following, the invention is described in more detail with reference to amino acid sequences, nucleic acid sequences and examples. However, no limitation of the invention is intended by the details of the examples. Rather, the invention pertains to any embodiment which comprises details which are not explicitly mentioned in the examples herein, but which the skilled person finds without undue effort.

### EXAMPLES:

### 1) Materials & Methods

### Palythoa culture:

*Palythoa* spp. including *Palythoa* aff. *clavata* are maintained in a culture system comprising six tanks (70 Liters each) plugged to a biological filter. The whole system is fed by artificial seawater (Instant Oceansalts, from Seachem Salinity) maintained at 26°C and exposed to a 12 h light/12 h dark photoperiod with an irradiance of 70 mol.quanta m⁻².s⁻¹. A skimmer has been set up to remove fatty acids and proteins from the system, and to avoid the accumulation of nitrogenous compounds that are toxic for marine invertebrates. The evaporation is compensated automatically by addition of DI-water. *Palythoa* spp. are fed on a daily basis with pellets made of fish meat (Formula One, from Ocean Nutrition) to maximize the growth rate.

### Symbiodinium dinoflagellate isolation:

Two extraction protocols have been implemented:
**Protocol (A):** Extractions from *Palythoa* aff. *clavata* is performed as described by Richier *et al.,* 2003 and Forcioli *et al.,* 2011. One gram of *Palythoa* aff. *clavata* is quickly dried out on paper to remove excess seawater, chopped into several pieces with a scalpel, suspended in a 50 mM phosphate buffer pH 7.8, 0.4 M sorbitol, and disrupted by syringe homogenization. The *Symbiodinium* dinoflagellate is separated from the extract by centrifugation at 3000 g for 5 min. The pellet was washed twice with the same extraction buffer, centrifuged at 3000 g for 3 min and then grounded in a mortar with liquid nitrogen. The resulting powder is resuspended in the same extraction buffer, and the solution filtered through a nylon mesh (100 µm) in order to eliminate as many skeleton residues as possible. The filtrate containing the *Symbiodinium* dinoflagellate is further applied on Petri dishes and cultured as described in the paragraph *"Symbiodinium* dinoflagellate culture".

**Protocol (B):** NaOH solution is used to extract the *Symbiodinium* dinoflagellate from *Palythoa* aff. *clavata.* Extracts are obtained by incubating 0.5 g of fresh animal previously dried out on paper and chopped into several pieces with a scalpel, in 500 µL of 1 M NaOH solution, at 37°C and during 1 h. The samples are vigorously shaken every 15 min during the NaOH treatment and extracts are centrifuged at 5000 g for 3 min. The *Symbiodinium* dinoflagellate pellets are washed twice with mill-Q water, neutralized with a 1 M HCl, resuspended in a F/2 culture medium, applied on Petri dishes and cultured as described in the paragraph *"Symbiodinium* dinoflagellate culture".

### Symbiodinium dinoflagellate culture:

The *Symbiodinium* dinoflagellate is routinely cultured in sterile conditions, in a one-Liter Erlenmeyer flask stoppered with a cotton wool, and containing a F/2 culture medium at pH 8.2 and incubated at 26.0 ± 0.1°C. The irradiance is about 100 µmol photons. m⁻². s⁻¹ (Sylvania Gro-Lux, Loessnitz, Germany), on a 12 h light/12 h dark photoperiod. A low stirring is performed with a magnetic stirrer.

### The F/2 culture medium is prepared as follows:

For one Liter of natural seawater, the composition of the F/2 culture medium comprises (final concentration): NaNO₃ 882 µM, NaH₂PO₄ H₂O 36 µM, and 1 mL of trace metal solution. The solution is further autoclaved at 120°C for 20 min, and after reaching ambient temperature, 0.5 mL of vitamin solution is added under sterile conditions.

The trace metal solution is as following (for 1 Liter of milli-Q water): FeCl₃ 6H₂O 3.15 g, Na₂EDTA 2H₂O 4.36 g, CuSO₄ 5H₂O 9.8 mg, Na₂MoO₄ 2H₂O 6.3 mg, ZnSO₄ 7H₂O 22.0 mg, CoCl₂ 6H₂O 10.0 mg, MnCl₂ 4H₂O 180 mg. The mixture is then autoclaved at 120°C for 20 min and stored at 4°C.

The vitamin solution is as following (for 1 Liter of milli-Q water): thiamine-HCl (vit. B₁) 200 mg, biotin (vit. H) 1 mg. Then, the solution is sterilized by passing on a 0.22 µm pore size filter (from Millipore) and stored at 4°C.

The *Symbiodinium* colonies are then isolated on Petri dish plates by adding 2.0% (w/v) of agar (from Difco, France) in the F/2 culture medium containing the trace elements, and autoclaved at 120°C during 20 min. The vitamin solution is added on the Petri dish plates at ambient temperature. The final extract containing the isolated *Symbiodinium* (see procedures described in the paragraph *"Symbiodinium* dinoflagellate isolation") is applied on the Petri dish plates which are further incubated at 26°C under a 12 h light/12 h dark photoperiod, until visualization of the colonies.

### Cell lines and culture media used for growth inhibitory effects:

The human cancer cell lines used that are sensitive to pro-apoptotic stimuli include the human Hs683 oligodendroglioma (Branle *et al.,* 2002; Ingrassia *et al.,* 2009) and the mouse B16F10 melanoma (Van Goietsenoven *et al.,* 2010) cell lines. The human cancer cell lines displaying various levels of resistance to pro-apoptotic stimuli include the human A549 non-small-cell lung (NSCLC) cancer (Mijatovic *et al.,* 2006) and the human U373n glioblastoma (Branle *et al.,* 2002) cell lines. The rodent cancer cell lines used include the mouse B16F10 melanoma (Van Goietsenoven *et al.,* 2010) and the 9L gliosarcoma (Lefranc *et al.,* 2002) cell lines. PLTX target preferentially the NaK pump which in rodents displays double mutation in the alpha-1 subunit with 100-1000 times decreases in sensitivity to NaK inhibitors like the cardenolide UNBS1450, when compared to human cells (Mijatovic *et al.,* 2007a and b; Lefranc *et al.,* 2008). Rodent cancer cell lines have thus been assayed to assess PLTX *in vitro* anticancer activity. The human HBL-100 epithelial cell line is also included. It is reported as a normal cell line, while it has been transformed by means of several oncogenes to render it immortal. Thus, from a biological point of view, this cell line cannot be considered as actually normal.

The cells are cultured at 37°C in sealed (airtight) Falcon plastic dishes (Gibco, Nunc, France) containing the medium, as indicated in **Table 2.** All media are supplemented with a mixture of glutamine (0.6 mg.mL⁻¹ final concentration; Gibco), penicillin (200 IU.mL⁻¹ final concentration; Gibco), streptomycin (200 IU.mL⁻¹ final concentration; Gibco), and 0.1 mg.mL⁻¹ gentamycin (Gibco). The FBS and FCS are previously heated for 1 h at 56 °C.

**Table 2: Cell lines and media**

| **Cell lines** | **Histological type** | **Cell culture media (Co)** | **Origin** |
|---|---|---|---|
| Hs683 | Oligodendroglioma (human) | RPMI + 10% FBS (LTI) | ATCC code HTB-138 |
| U373n | Glioblastoma (human) | RPMI + 10% FBS (LTI) | ECACC 08061901 |
| 9L | Gliosarcoma (rat) | RPMI + 10% FCS (LTI) | ATCC code CRL-2200 |
| B16F10 | Melanoma (mouse) | RPMI + 10% FBS (LTI) | ATCC code CRL-6475 |
| A549 | Lung carcinoma (human) | RPMI + 10% FBS (LTI) | DSMZ code ACC107 |
| HBL100 | Human epithelial transformed cells | RPMI + 10% FBS (LTI) | CLS code 330178 |
| NHDF | Normal human dermal fibroblast (juvenile foreskin) | MEM + 10% FBS (Gibco) | PC code c-12300 |
| MSC | Normal human fibroblast primocultures | FGM-2 BulletKit(Lonza) | Gift from Prof J. Dubois (*) |

| | | | |
|---|---|---|---|
| **ATCC**, American Type Culture Collection (Manassas, USA); **ECACC,** European Collection of Cell Cultures (Salisbury, UK); **DSMZ,** Deutsche Sammlung von Mikroorganismen und Zellkulturen (Braunschweig, Germany); **CLS,** Cell Line Services (Eppelheim, Germany); **PC,** PromoCell (Europe). **RPMI,** Roswell Park Memorial Institute (from **LTI,** Life Technologies-Invitrogen); **MEM,** Modified Eagle Medium (from Gibco); **FBS,** Fetal Bovine Serum; **FCS,** Fetal Calf Serum. *Address: Laboratoire de Chimie Analytique, Toxicologie et Chimie Physique Appliquée, Faculté de Pharmacie, Université Libre de Bruxelles (Belgium). | | | |

### Leukaemia cell lines and culture

Histiocytic lymphoma U937, chronic myelogenous leukaemia K562, T-cell leukaemia Jurkat were purchased from DSMZ (Braunschweig, Germany). MOLM14 was provided by .... Cells were cultured in RPMI 1640 or DMEM medium (Lonza, Verviers, Belgium) supplemented with 10 % (v/v) fetal calf serum (FCS; Lonza, Verviers, Belgium) and 1 % (v/v) antibiotic-antimycotic (BioWhittaker, Verviers, Belgium) at 37 °C and 5 % of CO2. Experiments were performed in culture medium containing 10% of FCS with cells in exponential growth phase. Cells were routinely controlled to exclude mycoplasma contamination. Non-adherent cells were seeded in fresh complete medium at concentrations of 300,000 cells/ml. After 1 h of recovery in the incubator, they were treated at the indicated concentrations.

### PLTX purification from Palythoa aff. clavata polyps:

One gram of fresh *Palythoa* aff. *clavata* polyps is gently detached from the stone are dried out on paper, chopped with a scalpel into several pieces and further placed in a 50 mL-Falcon tube containing 20 mL of 80% methanol in milli-Q water. After agitation during 10 h at 4°C with a magnetic stirrer and decantation, the liquid is removed with a glass pipette and is then centrifuged (8000 g, 20 min) or filtrated on a 0.45 µm PTFE membrane. The pellet is rinsed with water, centrifuged and the supernatants pooled. Methanol from the solution is then evaporated (rotavapor Buchi) and the resulting aqueous phase extracted twice with hexane or dichloromethane for removing pigments mainly composed of carotenoids. The aqueous phase is rapidly evaporated and deposited onto a two centimeters diameter glass column filled with 10 cm³ of C₁₈ reversed phase powder (Lichroprep® RP₁₈, from MERCK, code 1.09303.0100, France). The column is washed with acidified water (0.2% v/v formic acid), then with 50% MeOH in the same acidified water, the PLTX is finally eluted with 75% MeOH in acidified water. The yield of the purified PLTX is first evaluated by HPLC in this liquid fraction or by weighing the tube after evaporation to dryness of this fraction. Results are means of three determinations. Routinely, preparations containing 100 µg dry PLTX are kept at -20°C in sealed glass flasks. For *in vitro* growth inhibitory assays and videomicroscopy experiences, a stock solution of 0.01 M in dimethylsulfoxide is prepared, PLTX remaining stable over one month in this solution.

### PLTX purification from Symbiodinium dinoflagellate cells:

The *Symbiodinium* dinoflagellate culture is centrifuged at 3000 g at 4°C for 10 min. The supernatant is removed, the pellet resuspended with ice-cold milli-Q water and the solution further homogenized by sonication (ultrasonic Vibra-Cell equipment). Cell disruption is assessed by microscopy. After centrifugation of the extract at 12000 g for 10 min, the PLTX present in the supernatant is further purified by reverse phase chromatography on a Lichroprep® RP₁₈, as described previously.

### Compounds and treatment

All compounds were from SIGMA-ALDRICH (Bornem, Belgium) unless otherwise stated. Compounds were solubilized in dimethyl sulfoxide (DMSO) and stored at -20°C. Cycloheximide (CHX) was diluted in sterile water at 1 mg/ml and stored at -20°C. Cells were pre-treated before adding 10 µg/ml CHX. MG132 was diluted in DMSO at a stock concentration of 5 mM. For the proteasome activity assay, U937 cells were incubated at indicated concentrations for 2 h before the measurement (see section below). For protein stability assays, U937 cells were incubated for 6h before adding 5µM MG132. Samples were collected for further analysis at the indicated times. For the analysis of apoptosis parameters, cells were treated before adding 5 µM MG132 and samples were collected after 6 h. The pancaspase inhibitor I z-VAD (OMe)-FMK (zVAD; Calbiochem, Leuven, Belgium) was diluted in DMSO at a concentration of 50 mM and added 1 h before treatment at the final concentration of 50 µM.

### Molecular methods and phylogenetic analysis:

### DNA extraction:

In order to amplify both *Palythoa* and *Symbiodinium* genes, tissue sample collected for DNA extraction consisted of five tentacles joined by a small piece of polyp oral disc. Because symbiotic cells are much less abundant than coral cells, such a strategy results in a high concentration of *Symbiodinium* DNA compared to coral DNA, thus increasing the successful amplification of *Symbiodinium* genes. Genomic DNA is extracted using the Gentra Puregene Tissue Kit (Qiagen) according to the following modified protocol:

### Day 1

1. Cut 5 mm fresh tissue and place it into a 1.5 mL microcentrifuge tube,
2. Add 300 µL cell lysis solution,
3. Add 4 µL proteinase K and mix by inverting,
4. Incubate for 45 min at 65°C,
5. Incubate at 56°C overnight.

### Day 2

6. If tissue is not totally digested, add 4 µL proteinase K and incubate for 30 min at 65°C,
7. Add 100 µL protein precipitation solution and vortex for 20 s at high speed,
8. Incubate for 15 min at 4°C,
9. Centrifuge for 5 min at 13000 rpm,
10. Pipet 300 µL isopropanol into a clean 1.5 mL microcentrifuge tube and add the supernatant from the previous step by pouring carefully,
11. Mix by inverting 5-6 times,
12. Centrifuge for 5 min at 13000 rpm,
13. Carefully discard the supernatant, and drain the tube by inverting on a clean piece of absorbent paper, taking care that the pellet remains in the tube,
14. Add 300 µL of 70% ethanol and mix by inverting carefully,
15. Centrifuge for 5 min at 13000 rpm,
16. Carefully discard the supernatant, and drain the tube by inverting on a clean piece of absorbent paper, taking care that the pellet remains in the tube. Allow to air dry for up to 1 h,
17. Add 50 µL DNA hydration solution and mix by inverting,
18. Incubate at room temperature for 1 h to dissolve the DNA,
19. Mix by inverting and incubate at 4°C overnight,
20. Store at -20°C.

After extraction, DNA solution is diluted to a concentration of 40 ng.µL⁻¹ before the polymerase chain reaction (PCR).

### Gene amplification:

Two traditional DNA barcoding markers are used for genotyping the *Palythoa* species, one nuclear (ITS-rDNA) and the other mitochondrial (COI). ITS-rDNA sequence of *Palythoa* (18S ribosomal RNA gene, partial sequence; internal transcribed spacer 1, 5.8S ribosomal RNA gene, and internal transcribed spacer 2, complete sequence; 28S ribosomal RNA gene, partial sequence) of approximately 750-850 base pairs is amplified using the primers Zoanf-ITS (5' - CTT GAT CAT TTA GAG GGA GT - 3'; SEQ ID No: 1) and Zoanr-ITS (5' - CGG AGA TTT CAA ATT TGA GCT - 3'; SEQ ID No: 2). The PCR program is carried out according to the following parameters : an initial denaturing step at 94°C for 3 min, followed by 35 cycles of 1 min denature at 94°C, 1 min annealing at 50°C, 2 min extension at 72°C followed by 10 min extension at 72°C.

The portion of the mitochondrial COI gene of approximately 650 base pairs was also amplified for the *Palythoa,* with the following primers: HCO2198 (5' - TAA ACT TCA GGG TGA CCA AAA AAT CA - 3'; SEQ ID No: 3) and LCO1490 (5' - GGT CAA CAA ATC ATA AAG ATA TTG G - 3'; SEQ ID No: 4). PCR amplification is performed with the following parameters: an initial denaturing step at 94°C for 2 min followed by 5 cycles of 15 s denature at 92°C, 45 s annealing at 48°C and increasing of 24°C in 1 min, 1 min 30 extension at 72°C, followed by 30 cycles of 15 s denature at 92°C, 45 s annealing at 52°C, 45 s extension at 72°C, followed by 7 min extension at 72°C.

For genotyping the *Symbiodinium* clade living in the *Palythoa* tissues, ITS2-rDNA sequence (5.8S ribosomal RNA, partial sequence; internal transcribed spacer 2, complete sequence; 28S ribosomal RNA gene, partial sequence) of approximately 250-300 base pairs is amplified using the following specific primers ITS2-F1 (5' - GAA TTG CAG AAC TCC GTG - 3'; SEQ ID No: 5) and ITS2-R2 (5' - ATA TGC TTA AAT TCA GCG GGT - 3'; SEQ ID No: 6). PCR amplification is performed under stringent conditions for targeting the *Symbiodinium* genes instead of coral ones: an initial denaturing step at 94°C for 3 min followed by 12 cycles of 45 s denature at 94°C, 45 s annealing at 58°C and decreasing 0.5°C every cycle, 1 min extension at 72°C, followed by 20 cycles of 45 s denature at 94°C, 45 s annealing at 52°C and 1 min extension at 72°C followed by 7 min extension at 72°C.

PCR products are visualized by denaturing gradient gel electrophoresis. All fragments are sequenced in both directions using the amplicon primers.

### Phylogenetic analyses and DNA bar coding:

All sequences are first checked using NCBI BLAST then inspected by eye and aligned with orthologous sequences available in public Databases using CLUSTALW implemented in BioEdit 7.1.9 (http://www.mbio.ncsu.edu/bioedit/bioedit.html). Final alignments for phylogenetic reconstructions based on the *Symbiodinium* ITS2-rDNA sequences are done using Muscle (Edgar, 2004). For analyses of coral sequences, data from *Terrazoanthus, Hydrozaonthus* and *Parazoanthidae* genera are used as outgroups. Phylogenetic reconstructions based on *Symbiodinium* sequences are unrooted. Final alignments for both coral genes (ITS-rDNA and COI) and the single *Symbiodinium* gene (ITS2-rDNA) are 937, 462 and 332 nucleotides long respectively. Each data set is exported in Mega 5.1 (Tamura *et al.,* 2011) and the best-fit models of DNA evolution are searched using MODELTEST. The evolutionary model with the lowest Bayesian Information Criterion (BIC) is selected. Alignments are subjected to phylogenetic analyses with the maximum likelihood method (ML). Phylogenetic reconstructions are performed using 100 replicates with partial deletion of 75%. For *Symbiodinium* ITS2-rDNA data set, the distances are calculated using a Kimura's 2-parameters model incorporating a discrete gamma distribution with five categories (K2 + G5). For coral COI data set, a Kimura's 2-parameters model (K2) is used while for ITS-rDNA data set, a Kimura's 2-parameters model incorporating invariable sites with a discrete gamma distribution is used (K2 + G5 + I).

### HPLC control and quantification of the toxin:

To control the purity and quantify the purified PLTX, 2 µg of the sample diluted in milli-Q water are injected and analyzed by reverse-phase HPLC with a Waters equipment composed of a 1525 binary pump, a 2996 diode array detector, a Rheodyne injector (7725i model) fitted with a 20-µl loop, and a temperature control system. Files are acquired by the Empower software. Separations are carried out on a Symmetry C₁₈ reversed phase column (4.6 x 100 mm, ODS2, 5 µm) from Waters and protected with a guard cartridge. The elution is performed at 30°C at a flow rate of 0.8 ml/min and using a linear gradient of methanol (A) in acidified water with 0.2% (v/v) acetic acid (B), from 5 to 100% A during 20 min. PLTX is visualized at 263 nm and total spectra analyzed from 200 to 600 nm by using the Empower software (from Waters) in order to control the purity. For quantification, a standard calibration is established with commercial PLTX (from Wako Pure Chemical Industries, Japan). HPLC data are averages from two determinations.

### Mass spectrometry identification and control:

Matrix-assisted laser desorption ionization time-of-flight (MALDI-ToF) analysis are performed on a Microflex II mass spectrometer (Bruker, Germany). A 10 mg/mL solution of 2,5-dihydroxybenzoic (DHB) acid in 70/30 acetonitrile/water 0.1 % trifluoroacetic acid is used as matrix (Paz *et al.,* 2011). From a solution of 1 mg/mL of purified toxin in milli-Q water, 0.8 µL are mixed to 0.8 µL of the matrix solution. Mixtures are deposited as adroplet and allowed to dry at room temperature. Data are acquired in a positive reflectron mode, range is set from 600 to 5000 Da and pulsed ion extraction fixed to 150 ns. Ions formed upon irradiation by a smartbeam laser using a frequency of 200 Hz. The laser irradiance is set to 45-50% (relative scale 0-100) arbitrary units according to the corresponding threshold required for the applied matrix system. External mass calibration is done just before the acquisition of the sample using peptides calibration standard (Bruker Daltonics). Mass spectra are treated with the Flex Analysis software (Bruker, Germany) and no smoothing or baseline subtraction is done.

Purified PLTX is also analysed and controlled by ESI-MS/MS on a Q-ToF Synapt G1 High Definition mass spectrometer (Waters, UK) mounted with a nano spray ionization source and a V-mono-reflectron, in the positive mode. The following source settings are used: capillary voltage 3.2 kV, sampling cone 40 V, extraction cone 4 V, source temperature 120°C, desolvation gas flow 200 L.h⁻¹ (N2) at a temperature of 150°C, trap collision energy 38 eV. The calibration is performed with a solution of CsI 1 mg.mL⁻¹ and used in the 100-3200 mass range with a precision of +/- 3 ppm. A solution of purified PLTX (1 mg.mL⁻¹) in MeOH/H₂O (1:1) is diluted 25 times (15 µM final concentration) and injected via a syringe pump in the nano-source at a flow rate of 3 µL.min⁻¹.

### NMR control:

The complete chemical shift assignment of ¹H signals is compared to that of Kan *et al.* (2001). Deuterated methanol (CD₃OD) are chosen for it gave much sharper signals than in deuterated water (D₂O), as already observed (Kan *et al.,* 2001).

### MTT colorimetric assays for in vitro growth inhibitory effects:

The colorimetric MTT assay measures the number of metabolically active, living cells that are able to transform the yellow product 3-(4,5-dimethylthiazol-2-yl)-2,5-diphenyl tetrazolium bromide (MTT) into the blue formazan dye via mitochondrial reduction. The amount of formazan obtained at the end of the experiment, measured via spectrophotometry, is directly proportional to the number of living cells. Therefore, determination of the optical density enables a quantitative measurement of the effect of the investigated compound when compared with the control condition (untreated cells). The cell lines and culture media used are described in **Table 2.** To perform the assay, cells (5000 to 8000 cells/well depending on the cell line examined) are grown in a 96-well, flat-bottomed plate in 100 µL of media. Each cell line is seeded in its appropriate culture medium. Cells seeded are further treated for 72 h with different concentrations of purified PLTX ranging from 1 pM to 1 µM, with semilog concentration increasing. At the end of the incubation, blue formazan is solubilized with dimethylsulfoxide, and absorbance at 570 nm which is directly proportional to the number of living cells, is determined according to the method of Frédérick *et al.* (2012). The IC₅₀ concentrations are calculated from 96-well plates in which each experimental condition has been carried out in six replicates (one column of 6 wells). The control condition has been run two times in six replicates (two columns in the 96-well plates).

### Quantitative videomicroscopy:

Direct visualization of PLTX-induced effects on the cell proliferation and morphology of human Hs683 and U373n glioma cells is performed by means of time-lapse computer-assisted phase contrast microscopy, *i.e.* quantitative videomicroscopy. Specialized software packages are developed and implemented on computers connected to phase-contrast videomicroscopy systems placed in 37°C-heated incubators, as detailed by Debeir *et al.* (2008). Digitized images are acquired under a phase-contrast microscope (Olympus model IX50, with a 10:1 magnification ratio) using a digital 3.0M-PIXEL-USB 2.0 camera (model CG-032, Sharp Vision, China) driven by in-house-developed time-lapse software (Debeir *et al.,* 2008) with a resolution of 1024x768 pixels. An image is acquired every 4 min over a period of several hours. Movies are generated from these digitized images to enable a rapid viewing of the cell behavior for the duration of the experiments in control versus treated experimental conditions. Experiments are carried out in human Hs683 and U373n glioma cell lines in presence of PLTX at 0.01 and 1 nM. Each image represents the first image of each film that has been set up thanks to this approach for observation periods lasting from 3 to 22 h. One image is digitized every four minutes during the period of observation (thus 15 digitized images are recorded each hour) and the experiments are carried out in tetraplicate.

### 2) Results

### Morphological description of the PLTX-producing Palythoa:

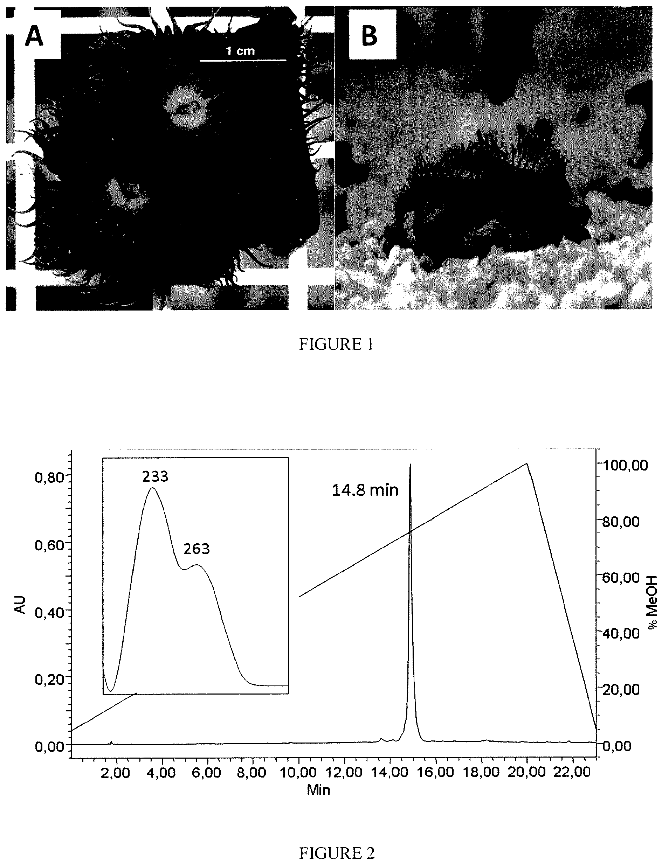

After the finding of a highly toxic *Palythoa* sp., the zoanthid and the toxin are identified. As seen in **Figure 1 (A)** and **(B)**, the *Palythoa* is small (1-2 cm wide), greenish solitary, anemone-like hexacorals with white stripes. The button-like body comprises a broad oral disc which is brown with green or blue overtones of 10-15 mm in diameter and a light center. It is surrounded by a short fringe of tentacles distributed in two distinct rows. The body and tentacules are punctuated with white dots. This zoanthid could correspond morphologically and genetically to a specimen recently described in Reimer *et al.* (2012). Phylogenetic analysis showed that it corresponds to *Palythoa* aff. *clavata* belonging to the family Sphenopidae from the Zoantharia order.

### Toxin purification and quantification:

The purification process performed to obtain pure PLTX from this *Palythoa* aff. *clavata* is described above in the section Materials & Methods.

Three experiments are done for evaluating the PLTX production yield by this zoanthidea. *Palythoa* aff. *clavata* is found to produce 2.81 ± 0.45 mg of PLTX/g wet polyp, as determined by HPLC. The high standard deviation recorded is probably due to the variable weight of the wet *Palythoa* aff. *clavata.* In any case, the recorded PLTX value (0.28% w/w) is the highest in regard to the literature (see **Table** 1) and corresponds to ten times more than the first value of 0.027% recorded by Moore and Sheuer (1971) for *Palythoa toxica.* In *Palythoa heliodiscus,* it has been found 1164 µg/g wet zoanthid for PLTX, and 3500 µg/g wet zoanthid for an analogue of PLTX, deoxy-PLTX (Deeds *et al.,* 2011).

### Chemical structure determination of the palytoxin:

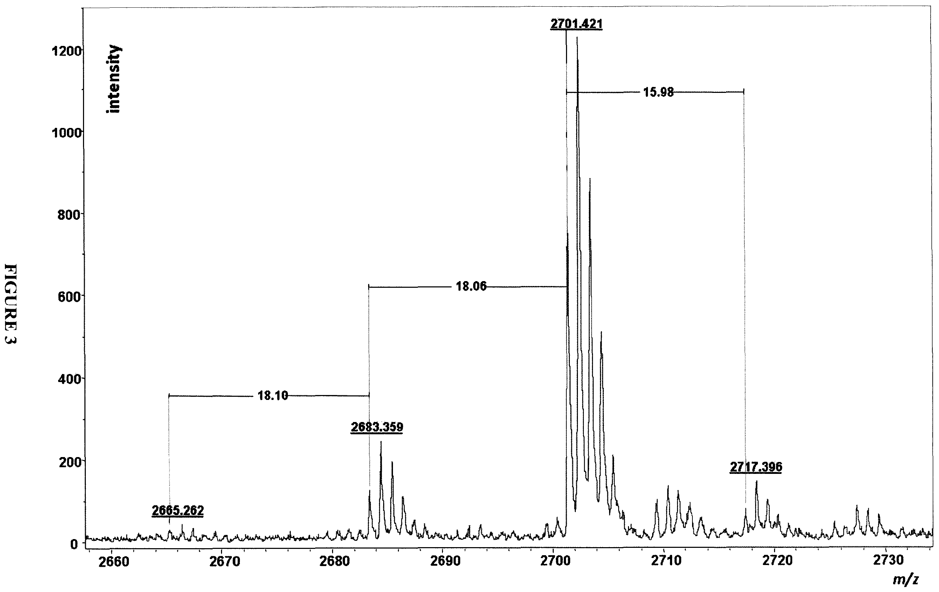

The purity of the obtained PLTX is controlled by DAD-HPLC all along the UV-visible range from 200 to 600 nm, no pigments or other compounds are detected even at low wavelengths. The purified toxin displayed two characteristic UV bands at 233 and 263 nm, as observed by Katikou (2007). In this case, PLTX is eluted as a symmetrical peak at within 80% methanol on our HPLC column (**Figure 2**), and its purity reached approximately 99% in regard to its mass spectra (**Figure 3**, and also Deeds *et al.* (2011); Ciminiello *et al.* (2011) that gave identical fragmentations) and ¹H-NMR data (data not shown, see Kan *et al.* (2001)).

By using the optimized matrix of Paz *et al.,* 2011, composed of 2,5-DHB in a 0.1% TFA/ACN mixture which gives better resolution than with a 2,5-DHB or HCCA (α-cyano-4-hydroxycinnamic acid) matrix, MALDI-ToF mass spectrometry in the positive mode shows single charged molecules containing mainly Na⁺ ions, and with a weak presence of K⁺ ions. The purified toxin shows a clear ion profile with a prominent ion at *m*/*z* 2701.421 [M + Na]⁺, thus identically to Paz *et al.,* and corresponding to a molecular mass of 2678.43 (**Figure 3**). Together with this main molecular ion, minor adducts at *m*/*z* 2683.359 [M + Na - H₂O]⁺, 2665.262 [M + Na - 2H₂O]⁺, and 2717.396 [M + K]⁺ are noted (**Figure 3**), confirming the high degree of purity of this toxin. ESI-tandem mass spectrometry analyses on a Q-ToF instrument are also performed to confirm the chemical structure of the purified PLTX, revealing a very complex ion pattern with multiply charged states (not shown). The cleavage between carbon 8 and 9 of the PLTX is demonstrated by the loss of the A moiety (see **Scheme 1**) which is recovered as a single fragment ion [M + H]⁺ *m*/*z* 327.126 in the full MS spectra, and corresponding to a C₁₆H₂₇N₂O₅ part formula generally observed in PLTX (data not shown). Other characteristic tri- and bi-charged ions are notably pointed at m/z 906.495 [M + 2H + K]³⁺, 1351.750 [M + H + Na]²⁺, and 1359.741 [M + H + K]²⁺ (data not shown). Further, MS/MS assignations confirmed the presence of a unique molecule and the chemical structure of the PLTX (Ciminiello *et al.,* 2011; Deeds *et al.,* 2011). The calculation based on these tri- and bi-charged ions, together with the MALDI-ToF data, attributed to the PLTX of *Palythoa* aff. *clavata* a molecular weight of 2678.48, thus similar to that of PLTX of *Palythoa tuberculosa.*

### Palythoa species identification (ITS-rDNA and COI genes):

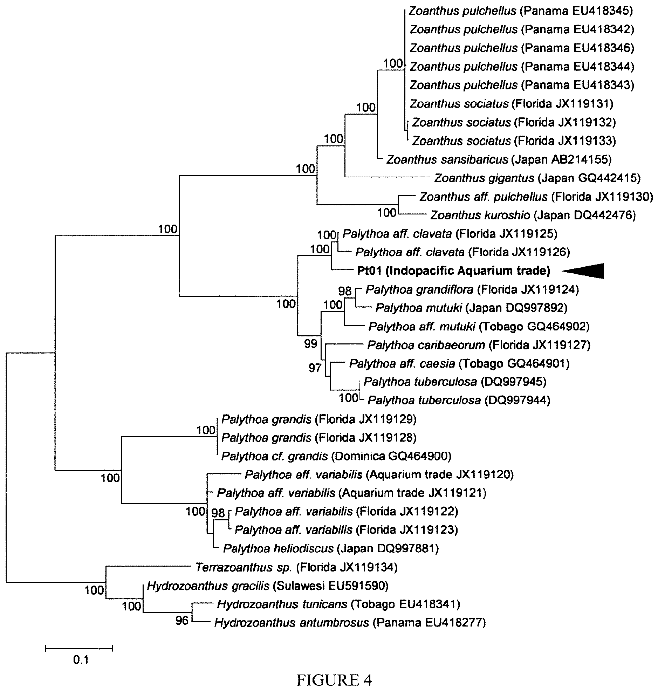

The *Palythoa* genus is paraphyletic in the tree based on the ITS-rDNA data set and only poorly recovered as monophyletic (bootstrap value, bv = 58) within that based on the COI mitochondrial gene. According to the ITS-rDNA phylogenetic reconstruction (**Figure 4**), two paraphyletic lineages of *Palythoa* are recovered with high support values, one contained *Palythoa grandis, Palythoa variabilis* and *Palythoa heliodiscus* (bv = 100) while the second consisted in a clade formed by *Palythoa* aff. *clavata, Palythoa caribaeorum, Palythoa mutuki, Palythoa caesia, Palythoa tuberculosa* and *Palythoa grandiflora* (bv = 100). The new sequence obtained from our specimen of *Palythoa* (Pt01) grouped within the latter as the sister group to *Palythoa* aff. *clavata* with high support value (bv = 100).

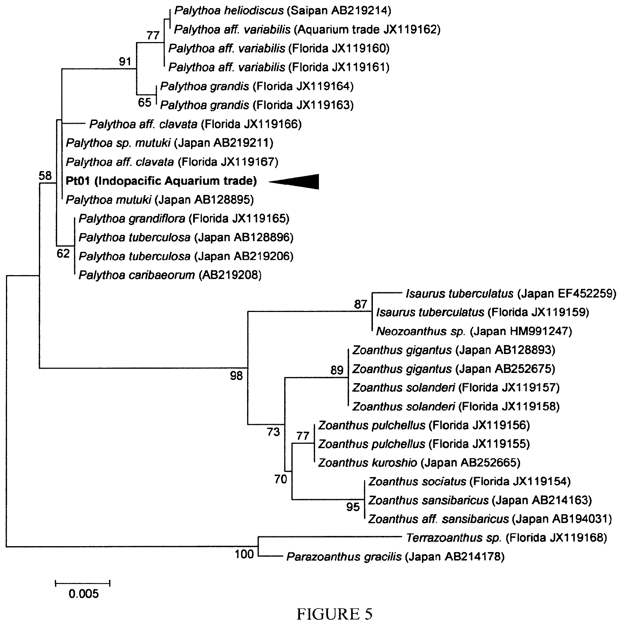

The COI gene analysis (**Figure 5**) provided a consensus tree less accurate than with the ITS-rDNA sequences. The monophyly of *Palythoa* is recovered but with low support (bv = 58) and only one of the two lineages highlighted in the ITS-rDNA is supported with high bootstrap value. Indeed, *Palythoa heliodiscus, Palythoa variabilis* and *Palythoa grandis* still show highly supported phylogenetic relationships (bv = 91), but the second *Palythoa* lineage is not supported because all the species belonging to this second clade appear in a paraphyletic assemblage. This concerns *Palythoa* aff. *clavata, Palythoa mutuki, Palythoa grandiflora, Palythoa tuberculosa, Palythoa caribaeorum* and Pt01.

### Symbiodinium clade typing (ITS2-rDNA gene):

Internal transcribed spacer 2 (ITS2-rDNA) typing results from *Symbiodinium* sp. are shown in **Figure 6**. After the sequencing step, the ITS2-rDNA sequence displayed clear chromatograms in both forward and reverse directions with no 'double-peaks' so no cloning step is performed to investigate a putative intragenomic variability and/or the presence of several *Symbiodinium* species.

Maximum likelihood analysis of ITS2-rDNA sequences isolated from various zooxanthellate metazoans including sponges, foraminiferans, molluscs and cnidarians as well as free-living dinoflagellates produced a well-resolved consensus tree in which the main *Symbiodinium* clades previously described in Cnidarians received high support values (bv = 93, 100, 87, 100, 100 and 95 for clades A-G respectively). The sequences from clade F, which is only described in foraminiferans, are not included. *Symbiodinium* ITS2-rDNA sequence isolated from the *Palythoa* specimen used in this study (ex Pt01) is unambiguously placed within the generalist clade C (bv = 87), within a large group that included many subclades of C previously observed in a variety of hosts, including isolates from other cnidarians such as scleractinians, octocorallians and zoanthids.

### Growth inhibitory effects in vitro and ICso determination:

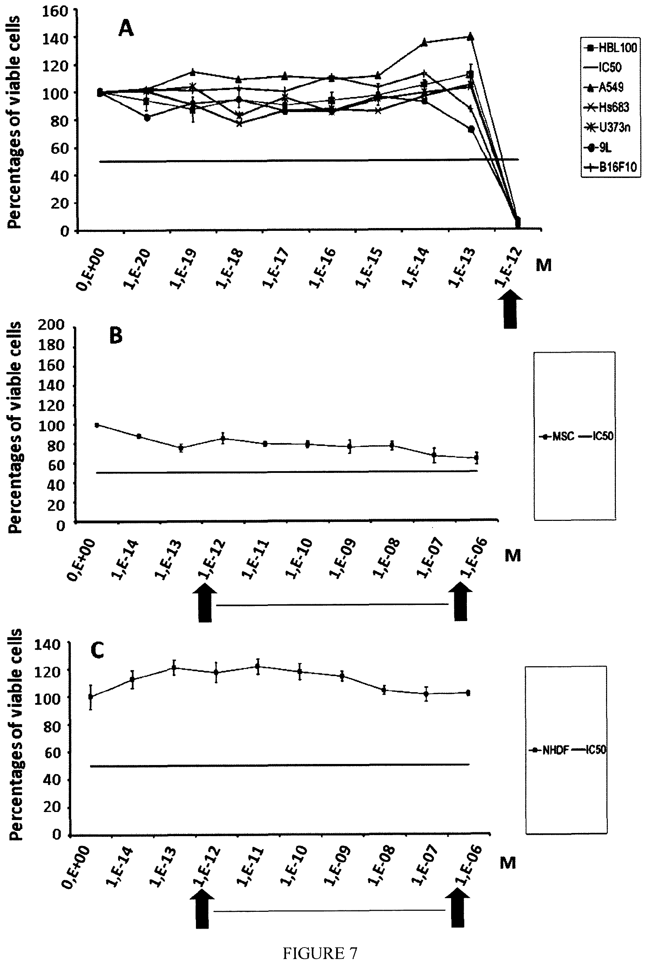

The MTT colorimetric assay-related data obtained are detailed in the **Table 3** and **Figure 7****.** The data reveal that the normal cells (the MSC and NHDF fibroblast cell lines) displayed ∼10⁶ lower in sensitivity in term of PLTX-mediated *in vitro* growth inhibition than cancer cells. The data also reveal that the HBL-100 transformed cell line behaved as a cancer, not as a normal cell line. The difference of ∼10⁶ lower sensitivity of normal cells in terms of PLTX-mediated *in vitro* growth inhibition when compared to cancer cells suggest that the main cellular target of PLTX is not the NaK if one refers to the data from Mijatovic *et al.* (2007a) and Lefranc *et al.* (2008).

The data further reveal that the rodent cell lines (surrounded in grey, see **Table 3**) display similar sensitivity to PLTX-mediated *in vitro* growth inhibition when compared to human cells, a feature that is once more in favor of the fact that the NaK would not be the primary/main cellular target of PLTX when exerting its *in vitro* growth inhibitory effects. Indeed, the IC₅₀ data given in **Table 3** must be compared to those obtained by Mijatovic *et al.* (2007a and b). The data obtained by means of the MTT colorimetric assay with PLTX in terms of *in vitro* growth inhibitory effects in human normal versus rodent and human cancer cell lines were highly reproducible. **Figure 7** (A, B and C) illustrates a graph (percentage viable cells versus PLTX concentration) obtained for calculation of the IC₅₀ for cancer and normal cells, after reading the 96-well plates including various types of cells treated for 72 h with PLTX (not shown).

**Table 3: IC₅₀ in vitro growth inhibitory concentration by 50% after having cultured the cells with PLTX for 72 h. For calculation of the mean, the IC₅₀ value for HBL-100 cell line is included here. In rodents (rat and mouse, in grey), the NaK displays double mutation in the alpha-1 subunit with 100-1000 times decrease in sensitivity to NaK inhibitors (Majatovic et al., 2007a and b; Lefranc et al., 2008). r, resistant to; s, sensitive to pro-apoptotic stimuli; pM, picomolar.**

| **Cell line** | **Pro-apoptotic stimuli** | **Normal / Cancer** | **Origin** | **IC₅₀ (in pM)** |
|---|---|---|---|---|
| **MSC** (fibroblast primoculture) | - | Normal | Human | >10 µM |
| **NHDF** (dermal fibroblast) | - | Normal | Human | >10 µM |
| **HBL-100** (mammary epithelial) | - | Normal but transformed cells for immortalization | Human | 0.645 |
| **A549** (lung carcinoma) | r | Cancer | Human | 0.665 |
| **Hs683** (glioma) | s | Cancer | Human | 0.575 |
| **U373n** (glioma) | r | Cancer | Human | 0.560 |
| **9L** (gliosarcoma) | - | Cancer | Rat | 0.390 |
| **B16F10** (melanoma) | s | Cancer | Mouse | 0.440 |
| Mean ± SEM | | | Human and murine | 0.545 ± 0.05 |

These morphological illustrations indicate that normal cells are less sensitive than cancer cells to the PLTX-mediated *in vitro* growth inhibitory effects, and that rodent cancer cells display similar sensitivity when compared to human cancer cells to these PLTX-mediated *in vitro* growth inhibitory effects.

Various data from the literature also report that ouabain-related *in vitro* IC₅₀ growth inhibitory effects are in the 10-100 nM ranges of concentration (Mijatovic *et al.,* 2007a and b), thus 10⁴ to 10⁵ higher than the < 10⁻¹² M (< 0.001 nM) IC₅₀ concentration evidenced by the Inventors for PLTX in various cancer cell lines.

### Growth inhibitory effects on cancer cells by videomicroscopy:

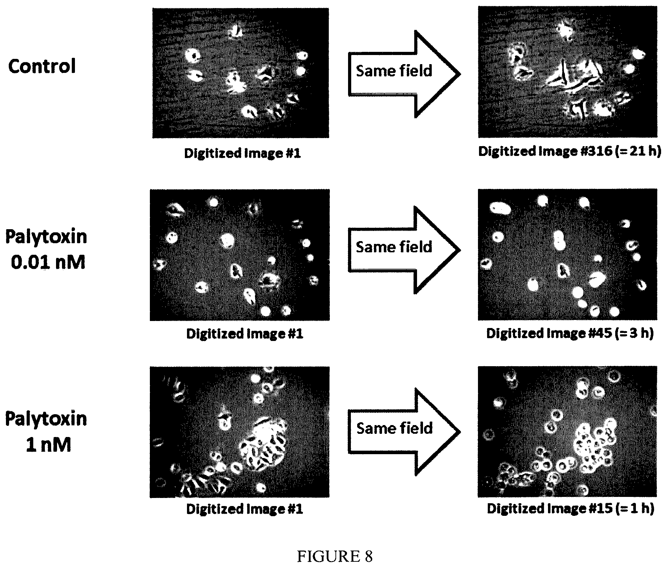

**Figure 8** illustrates the morphological pictures obtained with 0.01 and 1 nM PLTX on human Hs683 oligodendroglioma cells.

The concentration of 0.01 nM (*i.e.* 10 pM) represents -10 times the IC₅₀ *in vitro* growth inhibitory concentration associated with PLTX, while 1 nM represents -1000 times this IC₅₀ concentration (see **Table 3**). The IC₅₀ concentrations have been calculated after having cultured the various normal and cancer cells for 72 h with PLTX. **Figure 8** shows that Hs683 cells began to die about 3 h after having been treated with 0.01 nM, while all the Hs683 cells already died at 1 h post-treatment with 1 nM PLTX. The morphological appearances of Hs683 cells treated with 1 nM PLTX for 1 h are typical of cell swelling and bubbling occurring after the impairment of various ion channels, not only NaK.

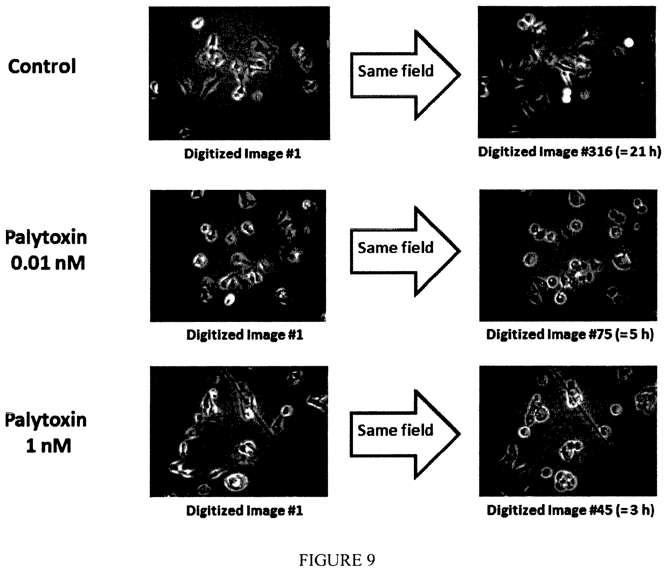

**Figure 9** illustrates the morphological pictures obtained with 0.01 and 1 nM PLTX on human U373n glioblastoma cells using experimental conditions identical to those used for analyzing the behavior of Hs683 oligodendroglioma cells (**Figure 8**). As for Hs683 glioma cells, the concentrations used, 0.01 and 1 nM, are -10 and -1000 times the IC₅₀ *in vitro* growth inhibitory concentration associated with PLTX (**Table 3**). **Figure 9** shows that U373n behaved as Hs683 when treated with PLTX, but with some delays. Indeed, U373n cells begin to die about 5 h (instead of 3 h for Hs683 cells; **Figure 8**) after having been treated with 0.01 nM PLTX, while all the U373n cells die 3 h (instead of 1 h for Hs683 cells; **Figure 8**) post-treatment with 1 nM PLTX.

The morphological appearances of U373n cells treated with 1 nM PLTX for 3 h are once more typical (as for Hs683 cells; **Figure 8**) of cell swelling and bubbling occurring after the impairment of various ion channels, not only NaK.

### Growth inhibitory effects on leukaemia cell lines in vitro and IC₅₀ determination:

PLTX was then further tested for the cell viability and proliferation on different human leukaemia cell lines corresponding to K562, JURKAT, U937 and MOLM14.

The results established that PLTX induces apoptosis in said leukaemia cell lines by down-regulating anti-apoptotic proteins, such as BID, MCL-1, BCL-2 and BCL-xL, via a proteasome-dependent degradation, and by inducing caspase activation (data not shown).

The data further reveal that in spite of the teaching of QUINN et al. (1974), PLTX reveals a strong growth inhibitory effect after a short time of culture, and this regardless of the human leukaemia cells lines used (Table 4).

**Table 4: IC₅₀ in vitro growth inhibitory concentration by 50% after having cultured the cells with PLTX for different time (from 2 to 8 h). pM, picomolar.**

| | | **IC₅₀ (in pM)** | | | | |
|---|---|---|---|---|---|---|
| **Leukaemia Cell line** | **Origin** | **2h** | **4h** | **6h** | **8h** | **10h** |
| **K562** | Human | 52.1 ± 5.85 | 32.8 ± 7.75 | 28.5 ± 7.28 | 24.9 ± 5.02 | Nd |
| **JURKAT** | Human | 12.8 ± 5.94 | 13.5 ± 2.13 | 8.82 ± 0.99 | 8.71 ± 1.00 | Nd |
| **U937** | Human | 24.8 ± 1.69 | 27.3 ± 8.14 | 14.0 ± 6.14 | 9.05 ± 0.71 | Nd |
| **MOLM14** | Human | Nd | Nd | Nd | Nd | 10.3 |

| | | | | | | |
|---|---|---|---|---|---|---|
| **Nd: not determined** | | | | | | |

Finally, the inventors thus established that the great potential of PLTX as an antitumor agent for the treatment of leukaemia. Simultaneously, the inventors confirms this potential by establishing a low PLTX toxicity on human PBMC with a maximum of 20% lethality at 100µM of PLTX (data not shown).

### In vivo safety and pharmacokinetics study:

Despite its potential toxicity, the toxicological profile of the molecule is poorly understood, with the exception of the work disclosed in Del Favero et al. (2013) using oral administration.

For estimating the toxicity of PLTX by other administration routes, mice were administrated with different doses either subcutaneously (s.c.) intravenously (i.v.) and their survival was determine five days post-injection.

**Table 5: mice survival after different PLTX doses (10 to 10,000 µg/kg) by s.c. or i.v. administration.**

| PLTX (ng/kg) | s.c. | i.v. |
|---|---|---|
| 10 | Nd | OK |
| 30 | OK | Nd |
| 100 | OK | Nd |
| 300 | OK | Nd |
| 1,000 | Nd | OK |
| 2,000 | OK | Nd |
| 10,000 | Death | Nd |

| | | |
|---|---|---|
| **Nd: not determined** | | |

The results show that no animal lethality was observed until a 10 µg/kg administration, the animal surviving without visible adverse effects to a 2 µg/kg PLTX administration (Table 5). These results are in agreement with those presented in Del Favero *et al.* (2013) establishing an estimation of the NOAEL (no observed adverse effect level) to an oral administration of 3 µg/kg/day of PLTX. This result was confirmed by s.c. daily PLTX administration for 15 days to mice with an initial 2 µg/kg administration (data not shown).

The PLTX pharmacokinetics was further determined by a s.c. PLTX administration of 2 µg/kg to two mice. Then, PLTX plasma concentration was determined at 30 min, 1h and 3 hours following PLTX injection.

**Table 6: plasma PLTX doses (10 to 10,000 µg/kg) by s.c. or i.v. administration.**

| Time following PLTX s.c. administration (h) | PLTX plasmatic concentration (nM) |
|---|---|
| 0.5 | 0.5-1 |
| 1 | 3-5 |
| 3 | 1-1.5 |

The results shows a maximal PLTX plasmatic concentration at 1h following the injection, which concentration rapidly decrease (table 6). This maximal plasmatic PLTX concentration corresponds to nearly 100 fold more than the efficient PLTX concentration on leukemic human cell lines.

### Xenografts and in vivo drug treatment assays:

NOD/SCID (nonobese diabetic/severe combined immunodeficient)/yc null mice (NSG) are obtained from CHARLES RIVER Laboratory.

The acute myeloid leukaemia luciferase-expressing U937 (0.2M in 100µL of PBS) cells are transplanted into caudal vein of mice (6-8 week-old male).

Next, control medium or increased concentration of PLTX (10, 50, 100, 250 ng/kg and 1 µg/kg) are injected subcutaneously to said mice, said administration being repeated 5 days a week until day 14 post injection.

Leukaemia expansion is followed in said mice by bioimaging using a Photon Imager (BIOSPACELAB).

Luciferin is injected i.p. (30mg/Kg in 100 µl PBS), followed by analysis 10 min after injection on anesthetized (3% isoflurane) animals. When the first leukaemia-related symptoms are observed (hunch-backed, a significant weight loss or ruffled coat), the mice are humanely euthanatized and survival rates is estimated by the Kaplan-Meier method.

**Plasmodium falciparum sensibility to PLTX:**The study is performed with on P. falciparum clones with distinct anti-malarial resistance patterns.

To evaluate rapidly Plasmodium falciparum growth in Vitro in the presence of PLTX, [3H]hypoxanthine is added to parasite microcultures and radioisotope incorporation is measured in the presence or absence of PLTX.

### Bibliographic references:

Apprill AM, RD Gates. 2007. Recognizing diversity in coral symbiotic dinoflagellate communities. Mol. Ecol. 16: 1127-1134.
Baker AC. 2003. Flexibility and specificity in coral-algal symbiosis: diversity, ecology and biogeography of Symbiodinium. Annu. Rev. Ecol. Evol. Syst. 34: 661-689.
Bellocci M, Sala GL, Prandi S 2011. The cytolytic and cytotoxic activities of palytoxin. Toxicon 57(3):449-59.
Béress L, J Zwick, HJ Kolkenbrock, PN Kaul, O Wassermann. 1983. A method for the isolation of the caribbean palytoxin (C-PTX) from the coelenterate (zooanthid) Palythoa caribaeorum. Toxicon 21: 285-290.
Berkelmans R, MJH van Oppen. 2006. The role of zooxanthellae in the thermal tolerance of corals: a 'nugget of hope' for coral reefs in an era of climate change. Proc. R. Soc. Lond. B 273: 2305-2312.
Branle F, F Lefranc, I Camby, J Jeuken, A Geurts-Moespot, S Sprenger, F Sweep, R Kiss R, I Salmon. 2002. Evaluation of the efficiency of chemotherapy in in vivo orthotopic models of human glioma cells with and without 1p/19q deletions and in C6 rat orthotopic allografts serving for the evaluation of surgery combined with chemotherapy. Cancer 95: 641-655.
Charlson AT, NA Zeliadt NA, EV Wattenberg. 2009. Extracellular signal regulated kinase 5 mediates signals triggered by the novel tumor promoter palytoxin. Toxicol Appl Pharmacol 241: 143-153.
Ciminiello P, C Dell'Aversano, E Fattorusso, M Forino, L Tartaglione, C Grillo, N Melchiorre. 2008. Putative palytoxin and its new analogue, ovatoxin-a, in Ostreopsis ovata collected along the Ligurian Coasts during the 2006 toxic outbreak. J. Am. Soc. Mass. Spectrom. 19: 111-120.
Ciminiello P, C Dell'Aversano, E DelloIacovo, E Fattorusso, M Forino, L Grauso, L Tartaglione, C Florio, P Lorenzon, M De Bortoli, A Tubaro, M Poli, G Bignami. 2009. Stereostructure and biological activity of 42-hydroxy-palytoxin: a new palytoxin analogue from Hawaiian Palythoa subspecies. Chem. Res. Toxicol. 22: 1851-1859.
Ciminiello P, C Dell'Aversano, E DelloIacovo, E Fattorusso, M Forino, L Tartaglione. 2011. LC-MS of palytoxin and its analogues: state of the art and future perspectives. Toxicon 57: 376-380.
Coffroth MA, SR Santos. 2005. Genetic diversity of symbiotic dinoflagellates in the genus Symbiodinium. Protist 156: 19-34.
Dawe CJ, Potter M. 1957. Morphologic and biologic progression of a lymphoid neoplasm of the mouse in vivo and in vitro. Am J Pathology. 33(3):603.
Debeir O, V Megalizzib, N Warzeea, R Kiss, C Decaesteckera. 2008. Videomicroscopic extraction of specific information on cell proliferation and migration in vitro. Exp. Cell Res. 314: 2985-2998.
Del Favero G, C Florio, B Codan, S Sosa, M Poli, O Sbaizero, J Molgo, A Tubaro, P Lorenzon. 2012. The stretch-activated channel blocker gd3+ reduces palytoxin toxicity in primary cultures of skeletal muscle cells. Chem Res Toxicol 25: 1912-1920.
Del Favero G, Beltramo D, Sciancalepore M, Lorenzon P, Coslovich T, Poli M, Testai E, Sosa S, Tubaro A. 2013. Toxicity of palytoxin after repeated oral exposure in mice and in vitro effects on cardiomyocytes. Toxicon. 75:3-15
Deeds JR, SM Handy, K White, JD Reimer. 2011. Palytoxin found in Palythoa sp. Zoanthids (Anthozoa, Hexacorallia) sold in the home aquarium trade. PLOS One 6: 1-9.
Edgar RC (2004). MUSCLE: multiple sequence alignment with high accuracy and high throughput. Nucleic acids research 32: 1792-1797.
Frédérick R, C Bruyere, C Vancraeynest, J Reniers, C Meinguet, L Pochet, A Backlund, B Masereel, R Kiss, J Wouters. 2012. Novel trisubstituted harmine derivatives with original in vitro anticancer activity. J. Med. Chem. 55: 6489-6501.
Forcioli D, PL Merle, C Caligara, M Ciosi, C Muti, P Francour, C Cerrano, D Allemand. 2011. Symbiont diversity is not involved in depth acclimation in the Mediterranean sea whip Eunicella singularis. Mar. Ecol. Prog. Ser. 439: 57-71.
Gleibs S, D Mebs, B Weerding. 1995. Studies on the origin and distribution of palytoxin in a Caribbean coral reef. Toxicon 33: 1531-1537.
Görögh T, L Bèress, ES Quabius, P Ambrosch, M Hoffmann. 2013. Head and neck cancer cells and xenografts are very sensitive to palytoxin: decrease of c-jun n-terminal kinase-3 expression enhances palytoxin toxicity. Molecular Cancer 12: 1-11.
Guillard RRL, JH Ryther. 1962. Studies of marine planktonic diatoms. I. Cyclotella nana Hustedt and Detonula confervacea Cleve. Can. J. Microbiol. 8: 229-239.
Hilgeman DW. 2003. From a pump to a pore: how palytoxin opens the gates. Proc Natl Acad Sci USA 100: 386-388.
Iglesias-Prieto R, RK Trench. 1997. Photoadaptation, photoacclimation and niche diversification in invertebrate-dinoflagellate symbioses. Pp. 1319-1324 in Proceedings of the 8th International Coral Reef Symposium, Vol. 2, H. A. Lessios and I. G. Macintyre, eds. Smithsonian Tropical Research Institute, Panama.
Ingrassia L, F Lefranc, J Dewelle, L Pottier, V Mathieu, S Spiegl-Kreinecker, S Sauvage, M El Yazidi, M Dehoux, W Berger, E Van Quaquebeke, R Kiss. 2009. Structure-activity-relationship analysis of novel derivatives of narciclasine (an Amaryllidaceae isocarbostyril alkaloid) as potential anti-cancer agents. J Med Chem 52: 1100-1114.
Johannes RE, WJ Wiebe. 1970. Method for determination of coral tissue biomass and composition. Limnol. Oceanogr. 15: 822-824.
Kan Y, D Uemura, Y Hirata, M Ishiguro, T Iwashita. 2001. Complete NMR signal assignment of palytoxin and N-acetylpalytoxin. Tetrahedron Lett 42: 3197-3202.
Katikou P. 2007. Chemistry of palytoxins and ostreocins. In: Phycotoxins, Chemistry and Biochemistry; Botana LM Ed; Blackwell Publishing: Ames, IA, USA, pp 75-93.
Kerbrat AS, Z Amzil, R Pawlowiez, S Golubic, M Sibat, HT Darius, M Chinain, D Laurent. 2011. First evidence of palytoxin and 42-hydroxy-palytoxin in the marine cyanobacterium Trichodesmium. Mar. Drugs 9: 543-560.
Kimura S, K Hashimoto. 1973. Purification of the toxin in a zoanthid Palythoa tuberculosa. Publ. Seto. Mar. Biol. Lab. 20: 713-718.
Lefranc F, I Camby, N Belot, E Bruyneel, C Chaboteaux, J Brotchi, M Mareel, I Salmon, R Kiss. 2002. Gastrin significantly modifies the migratory abilities of experimental glioma cells. Lab Invest 82: 1241-1252.
Lefranc F, T Mijatovic, Y Kondo, S Sauvage, I Roland, D Krstic, V Vasic, P Gailly, S Kondo, G Blanco, R Kiss. 2008. Targeting the alpha-1 subunit of the sodium pump (the Na+/K+-ATPase) to combat glioblastoma cells. Neurosurgery 62: 211-222.
Lenoir S, L Ten-Hage, J Turquet, JP Quod, C Bernard, MC Hennion. 2004. First evidence of palytoxin analogues from an Ostreopsis mascarenensis (Dinophyceae) benthic bloom in southwestern Indian Ocean. J. Phycol. 40: 1042-1051.
Louzao MC, IR Ares, E Cagide. 2008. Marine toxins and the cytoskeleton: a new view of palytoxin toxicity. FEBS J 275: 6067-6074.
Louzao MC, IR Ares, E Cagide, B Espina, N Vilarino, A Alfonso, MR Vieytes, LM Botana. 2011. Palytoxins and cytoskeleton: An overview. Toxicon 57:460-469.
Mijatovic T, V Mathieu, JF Gaussin, N De Neve, F Ribaucour, E Van Quaquebeke, P Dumont, F Darro, R Kiss. 2006. Cardenolide-induced lysosomal membrane permeabilization contributes therapeutic benefits in experimental human non-small-cell-lung cancers. Neoplasia 8: 402-412. a-Mijatovic T, I Roland, E Van Quaquebeke, B Nilsson, A Mathieu, F Van Vynckt, F Darro, G Blanco, V Facchini, R Kiss. 2007. The alpha-1 subunit of the sodium pump could represent a novel target to combat non-small cell lung cancers. J Pathol 212: 170-179.
b-Mijatovic T, E Van Quaquebeke, B Delest, O Debeir, F Darro, R Kiss. 2007. Cardiotonic steroids on the road to anti-cancer therapy. BBA Rev Cancer 1776: 32-57.
Moore RE, PJ Scheuer. 1971. Palytoxin, new marine toxin from a Coelenterate. Science 172: 495-498.
Morera C, MA Villanueva. 2009. Heat treatment and viability assessment by Evans blue in cultured Symbiodinium kawagutii cells. World J. Microbiol. Biotechnol. 25: 1125-1128.
Oku N, NU Sata, S Matsunaga, H Uchida, N Fusetani. 2004. Identification of palytoxin as a principle which causes morphological changes inrat 3Y1 cells in the zoanthid Palythoa aff. margaritae. Toxicon 43: 21-25.
Paz B, P Riobo, JM Franco. 2011. Preliminary study for rapid determination of phycotoxins in microalgae whole cells using matrix-assisted laser desorption ionization time-of-flight mass spectrometry. Rapid Commun. Mass Spectrom. 25: 3627-3639.
Pelin M, Boscolo S, Poli M, Sosa S, Tubaro A, C Florio. 2013. Characterization of palytoxin binding to HaCaT cells using a monoclonal anti-palytoxin antibody. Mar. Drugs 11: 584-598.
Pey A, T Zamoum, D Allemand, P Furla, PL Merle. 2011. Depth dependant thermotolerance of the symbiotic Mediterranean gorgonian Eunicella singularis: evidence from cellular stress markers. J. Exp. Mar. Biol. Ecol. 404: 73-78.
Pochon X, RD Gates. 2010. A new Symbiodinium clade (Dinophyceae) from soritid foraminifera in Hawai'i. Mol. Phylogenet. Evol. 56: 492-497.
Quinn RJ, M Kashiwagi, RE Moore, TR Norton. 1974. Anticancer activity of zoanthids and the associated toxin, palytoxin, against ehrlich ascites tumor and p-388 lymphocytic leukaemia in mice. J. Pharmac Sci 63: 257-260.
Ramos V, V Vasconcelos. 2010. Palytoxin and analogs: Biological and ecological effects. Mar. Drugs 8: 2021-2037.
Reimer JD, C Foord, Y Irei. 2012. Species Diversity of Shallow Water Zoanthids (Cnidaria: Anthozoa: Hexacorallia) in Florida. J. Marine Biol. 2012: 1-14.
Richier S, PL Merle, P Furla, D Pigozzi, F Sola, D Allemand. 2003. Characterization of superoxide dismutases in anoxia- and hyperoxia-tolerant symbiotic cnidarians. Biochim. Biophys. Acta 1621: 84-91.
Rodrigues AM, ACG Almeida, AFC Infantosi. 2008. Effect of palytoxin on the sodium-potassium pump: model and simulation. Phys Biol 5: 036005.
Rossi R, V Castellano, E Scalco, L Serpe, A Zingone, V Soprano. 2010. New palytoxin-like molecules in Mediterranean Ostreopsis cf. ovata (dinoflagellates) and in Palythoa tuberculosa detected by liquid chromatography-electrospray ionization time-of-flight mass spectrometry. Toxicon 56: 1381-1387.
Rossini GP, A Bigiani. 2011. Palytoxin action on the Na+,K+-ATPase and the disruption of ion equilibria in biological systems. Toxicon 57: 429-439.
Rowan R. 2004. Coral bleaching: thermal adaptation in reef coral symbionts. Nature 430: 742.
Santiago-Vazquez L, Ranzer L, R Kerr. 2006. Comparison of two total RNA extraction protocols using the marine gorgonian coral Pseudopterogorgia elizabethae and its symbiont Symbiodinium sp. Electron. J. Biotechnol. 9: doi:10.2225/vol9issue5-fulltext-15.
Satoh E, T Ishii T, M Nishimura. 2003. Palytoxin-induced increase in cytosolic-free Ca2+ in mouse spleen cells. Eur J Pharmacol 465: 9-13.
Staquet MJ, Byar DP, Green SB, Rozencweig M. 1983. Clinical predictivity of transplantable tumor systems in the selection of new drugs for solid tumors: rationale for a three-stage strategy. Cancer Treat Rep; 67:753-765.
Stat M, E Morris, RD Gates. 2008. Functional diversity in coral-dinoflagellate symbiosis. Proc. Natl. Acad. Sci. 105: 9256-9261.
Stat M, RD Gates. 2011. Clade D Symbiodinium in scleractinian corals: a "nugget" of hope, a selfish opportunist, an ominous sign, or all of the above? J. Mar. Biol. 2011: e730715.
Tamura K, D Peterson, N Peterson, G Stecher, M Nei, S Kumar (2011). MEGA 5: Molecular Evolutionary Genetics Analysis using Maximum Likelihood, Evolutionary Distance, and Maximum Parsimony Methods. Molecular Biology and Evolution 28: 2731-2739.
Taniyama S, O Arakawa, M Terada, S Nishio, T Takatani, Y Mahmud, T Noguchi. 2003. Ostreopsis sp., a possible origin of palytoxin (PTX) in parrot fish Scarusovifrons. Toxicon 42: 29-33.
Tchernov D, MY Gorbunov, C de Vargas, S Narayan Yadav, AJ Milligan, M Haggblom, PG Falkowski. 2004. Membrane lipids of symbiotic algae are diagnostic of sensitivity to thermal bleaching in corals. Proc. Natl. Acad. Sci. 101: 13531-13535.
Uemura D, Y Hirata, T Iwashita, H Naoki. 1985. Studies on palytoxins. Tetrahedron 41: 1007-1117.
Ukena T, M Satake, M Usami, Y Oshima, H Naoki, T Fujita, Y Kan, T Yasumoto. 2001. Structure elucidation of ostreocin d, a palytoxin analog isolated from the dinoflagellate Ostreopsis siamensis. Biosci. Biotechnol. Biochem. 65: 2585-2588.
Valverde I, J Lago, A Reboreda, JM Vieites, AG Cabado. 2008. Characteristics of palytoxin-induced cytotoxicity in neuroblastoma cells. Toxicol. In Vitro 22: 1432-1439.
Van Goietsenoven G, J Hutton, JP Becker, B Lallemand, F Robert, F Lefranc, C Pirker, G Vandenbussche, P Van Antwerpen, A Evidente, W Berger, M Prévost, J Pelletier, R Kiss, TG Kinzy, A Kornienko, V Mathieu. 2010. Targeting of eEF1A with Amaryllidaceae isocarbostyrils as a strategy to combat melanomas. FASEB J 24: 4575-4584.
Warner ME, WKFitt, GW Schmidt. 1999. Damage to photosystem II in symbiotic dinoflagellates: a determinant of coral bleaching. Proc. Natl. Acad. Sci. 96: 8007-8012.
Wattenberg EV. 2011. Modulation of protein kinase signaling cascades by palytoxin. Toxicon 57: 440-448.
Wiles JS, JA Vick, MK Christensen. 1974. Toxicological evaluation of palytoxin in several animal species. Toxicon 12: 427-433.
Yasumoto T, M Murata. 1990. Polyether toxins involved in seafood poisoning. In: Hall S, G Strichartz (Eds.), Marine Toxins. ACS Symposium Series 418. American Chemical Society, pp. 120-132.
Zamoum T, P Furla. 2012. Methods and techniques : Symbiodinium isolation by NaOH treatment. J. Exp. Biol 215: 3875-3880.

## Claims

1. A pharmaceutical composition for use in a method of preventing and/or treating leukaemia in a human, said composition comprising palytoxin (PLTX) and a pharmaceutically acceptable carrier.

2. The pharmaceutical composition for the use of claim 1, wherein the route of administration of said composition is parenteral.

3. The pharmaceutical composition for the use of claim 2, wherein the route of administration is intravenous and/or subcutaneous administration.

4. The pharmaceutical composition for the use of any one of claim 1 to 3, wherein said leukaemia is a lymphoblastic or a myelogenous leukaemia.

5. The pharmaceutical composition for the use of claim 4, wherein said leukaemia is a myelogenous leukaemia.

6. The pharmaceutical composition for the use of any one of claim 1 to 5, wherein said PLTX is administrated between 10 ng/kg and 2 µg/kg.

7. The pharmaceutical composition for the use of claim 6, wherein said PLTX is administrated between 20 ng/kg and 1 µg/kg;

8. The pharmaceutical composition for the use of claim 7, wherein said PLTX is administrated between 50 ng/kg and 0.5 µg/kg.

9. The pharmaceutical composition for the use of any one of claim 1 to 8, wherein said PLTX is obtained from Palythoa aff. clavata polyps.

10. The pharmaceutical composition for the use of claim 9, wherein the Palythoa aff. clavata comprises the Symbiodinium dinoflagellate.

## Patentansprüche

1. Pharmazeutische Zusammensetzung zur Verwendung in einem Verfahren zur Verhütung und/oder Behandlung von Leukämie bei einem Menschen, wobei die Zusammensetzung Palytoxin (PLTX) und einen pharmazeutisch unbedenklichen Träger umfasst.

2. Pharmazeutische Zusammensetzung zur Verwendung nach Anspruch 1, wobei der Verabreichungsweg der Zusammensetzung parenteral ist.

3. Pharmazeutische Zusammensetzung zur Verwendung nach Anspruch 2, wobei der Verabreichungsweg der Zusammensetzung intravenös und/oder subkutan ist.

4. Pharmazeutische Zusammensetzung zur Verwendung nach einem der Ansprüche 1 - 3, wobei es sich bei der Leukämie um eine lymphoblastische oder eine myeloische Leukämie handelt.

5. Pharmazeutische Zusammensetzung zur Verwendung nach Anspruch 4, wobei es sich bei der Leukämie um eine myeloische Leukämie handelt.

6. Pharmazeutische Zusammensetzung zur Verwendung nach einem der Ansprüche 1 - 5, wobei das PLTX in einer Menge zwischen 10 ng/kg KG und 2 µg/kg KG verabreicht wird.

7. Pharmazeutische Zusammensetzung zur Verwendung nach Anspruch 6, wobei wobei das PLTX in einer Menge zwischen 20 ng/kg KG und 1 µg/kg KG verabreicht wird

8. Pharmazeutische Zusammensetzung zur Verwendung nach Anspruch 7, wobei wobei das PLTX in einer Menge zwischen 50 ng/kg KG und 0,5 µg/kg KG verabreicht wird

9. Pharmazeutische Zusammensetzung zur Verwendung nach einem der Ansprüche 1 - 8, wobei das PLTX aus Polypen der Gattung Palythoa aff. clavata gewonnen wird.

10. Pharmazeutische Zusammensetzung zur Verwendung nach Anspruch 9, wobei die Palythoa aff. clavata das Dinoflagellat Symbiodinium umfasst.

## Revendications

1. Composition pharmaceutique pour une utilisation dans une méthode de prévention et/ou de traitement d'une leucémie chez un humain, ladite composition comprenant de la palytoxine (PLTX) et un véhicule pharmaceutiquement acceptable.

2. Composition pharmaceutique pour une utilisation selon la revendication 1, dans laquelle la voie d'administration de ladite composition est parentérale.

3. Composition pharmaceutique pour une utilisation selon la revendication 2, dans laquelle la voie d'administration est une administration intraveineuse et/ou sous-cutanée.

4. Composition pharmaceutique pour une utilisation selon l'une quelconque des revendications 1 à 3, dans laquelle ladite leucémie est une leucémie lymphoblastique ou myéloïde.

5. Composition pharmaceutique pour une utilisation selon la revendication 4, dans laquelle ladite leucémie est une leucémie myéloïde.

6. Composition pharmaceutique pour une utilisation selon l'une quelconque des revendications 1 à 5, dans laquelle ladite PLTX est administrée à raison d'entre 10 ng/kg et 2 µg/kg.

7. Composition pharmaceutique pour une utilisation selon la revendication 6, dans laquelle ladite PLTX est administrée à raison d'entre 20 ng/kg et 1 µg/kg.

8. Composition pharmaceutique pour une utilisation selon la revendication 7, dans laquelle ladite PLTX est administrée à raison d'entre 50 ng/kg et 0,5 µg/kg.

9. Composition pharmaceutique pour une utilisation selon l'une quelconque des revendications 1 à 8, dans laquelle ladite PLTX est obtenue à partir de polypes Palythoa aff. clavata.

10. Composition pharmaceutique pour une utilisation selon la revendication 9, dans laquelle le Palythoa aff. clavata comprend le Symbiodinium dinoflagellate.
